# EUROPEAN PATENT APPLICATION

(11) **EP 4 464 381 A1**
(43) Date of publication of application: **20.11.2024**
(21) Application number: 24173756.8
(22) Date of filing: 02.05.2024
(51) Int. Cl.: A61P 35/00, C07K 16/28

(54) **MICA ANTIBODY WITH AFFINITY MATURATION AND USE THEREOF**

(30) Priority: 06.05.2023 CN 202310509684
(71) Applicant: Hefei TG Immunopharma Co., Ltd., Hefei, Anhui 230001 (CN)
(72) Inventor: CAO, Guoshuai, Hefei, Anhui, 230001 (CN); CHENG, Ying, Hefei, Anhui, 230001 (CN); LI, Yangyang, Hefei, Anhui, 230001 (CN); WU, Yuwei, Hefei, Anhui, 230001 (CN)
(74) Representative: Latscha Schöllhorn Partner AG

(57) **Abstract**

Provided are an antibody or antigen-binding fragment thereof, and application thereof. The antibody or the antigen-binding fragment thereof includes: heavy chain variable region CDR1, CDR2 and CDR3 sequences as set forth in SEQ ID NO: 1, SEQ ID NO: 2, and SEQ ID NO: 3 or as set forth in amino acid sequences having at least 80% identity to SEQ ID NO: 1, SEQ ID NO: 2, and SEQ ID NO: 3; and light chain variable region CDR1, CDR2 and CDR3 sequences as set forth in SEQ ID NO: 4, SEQ ID NO: 5, and SEQ ID NO: 6 or as set forth in amino acid sequences having at least 80% identity to SEQ ID NO: 4, SEQ ID NO: 5, and SEQ ID NO: 6. The antibody or the antigen-binding fragment thereof can bind to human and monkey MICA proteins at high affinity, thereby facilitating an effect of peripheral blood mononuclear cells (PBMC) for killing tumors.

## Description

### FIELD

The present disclosure relates to the field of antibodies. Particularly, the present disclosure relates to a major histocompatibility complex class I chain related polypeptide A (MICA) antibody with affinity maturation and uses thereof, and more particularly, to an antibody or antigen-binding fragment thereof, a nucleic acid molecule, an expression vector, a method for preparing the antibody or antigen-binding fragment thereof, a recombinant cell, a composition and a use thereof, and a medicament and a use thereof.

### BACKGROUND

Cancer is a major disease affecting survival and development of mankind. The latest data indicate that the number of new cases of cancer is up to about 19 million every year globally, and that the number of cancer-caused death cases is up to about 10 million per year. Th incidence and mortality of cancers exhibit an increasing trend.

Except for surgical removal, the conventional cancer treatment methods such as chemotherapy and radiotherapy have disadvantages of significant side effects and easy recurrence. In recent years, immunotherapy, including tumor-targeted antibodies, immune checkpoint antibodies, and bispecific antibodies, has become a new focus and a promising approach against cancers.

In recent years, the immunotherapy represented by PD-1/L1 exhibits huge potential. However, a nonnegligible fact is in that more patients cannot benefit from the therapy even the total response ratio of the currently approved PD-1/L1 therapy with the most extensive indications is up to 30%. An immune checkpoint molecule is an inhibitory molecule expressed on the surface of immune cells including T cells, NK cells, and mononuclear macrophages, capable of transmitting an inhibitory signal into the immune cells after binding to a corresponding ligand, thereby inhibiting the immune cells' functions against cancer. On the one hand, since immune checkpoint ligand expressions of tumors and tumor infiltrating lymphocytes have extremely high heterogeneity, the immune checkpoint therapy of single species is not universally suitable for all patients, and thus most of the patients cannot benefit from the immune checkpoint therapy. On the other hand, the patients who have accepted the immune checkpoint therapy may have recurrent tumors and have tolerance to the immune checkpoint therapy, such that continuous therapy may have no curative effects. In addition, T cells recognize neo-antigens, i.e., tumor gene mutation antigens, through T cell receptors (TCR) on the surfaces. However, some tumors, refer to as "cold tumors", have a low frequency in gene mutation and thus have small varieties of neo-antigens. The existing immune checkpoint therapy such as the PD-1/L1 therapy aims to recover T cells' own function for purpose against cancer. In this regard, T cells cannot effectively recognize the "cold tumors". Therefore, the immune checkpoint therapy is ineffective to the cold tumors.

NK cells are very important anticancer immune cells of another type. NK cells have different recognition mechanisms from T cells. A series of activating receptors is expressed on the surfaces of the NK cells, and the activating receptors recognize and bind to ligands expressed on the tumor surfaces. For example, NK cells recognize and bind to ligands such as maj or histocompatibility complex class I chain related polypeptides A and B (MICA and MICB) on the tumor surfaces through an activating receptor NKG2D, and then tumor cells are activated and killed.

MICA and MICB are proteins encoded by autologous genes and are not expressed in normal tissues or cells. When the cells become tumor cells through malignant transformation, MICA and MICB are expressed on the surfaces of the tumor cells. The NK cells can recognize and kill the tumors through NKG2D-MICA/B interaction.

Tumors may down-regulate MICA/B expressions (including matrix metalloproteinase-mediated MICA/B shedding) on the cell surfaces through various mechanisms. MICA and MICB are type I transmembrane protein, whose extracellular domains include three domainsα1, α2 and α3. MICA and MICB are bound to NKG2D through the domains α1 and α2. Under the effects of matrix metalloproteinase, most of the amino acids of the extracellular domains of MICA/B (including the domains α1 and α2 and a part of the domain α3) shedding from the surfaces of the tumor cells. The NKG2D cannot bind to the one part of the domain α3 remaining on the cell surface. Therefore, the tumors can escape from immune surveillance of the NK cells.

Research indicates that, a MICA antibody capable of binding to the domain α3 can inhibit MICA shedding from the tumor surface and further facilitate NKG2D-MICA/B-mediated NK cell recognition, thereby inhibiting tumor immune escape. Up to now, only one monoclonal MICA antibody medicament (CLN-619) enters a phase I clinical stage. Therefore, it is of great significance for tumor treatment in developing MICA-targeted monoclonal antibody medicament.

### SUMMARY

An object of the present disclosure is to solve at least one of technical problems existing in the related to some extent at least.

The present disclosure is based on the following problems and facts discovered by the Applicant.

MICA is not expressed in normal tissues of the majority of people and has low expression in tissues such as the ovary and testis only. However, an extensive high expression of MICA can be found in tumor tissues, enabling MICA to become a potential tumor therapeutic target. Up to now, only one monoclonal MICA antibody, i.e., CLN-619, is available in the clinical test stage. In addition, Genentech has developed a MICA antibody (1D5V11 as mentioned in the present disclosure is disclosed in a Genentech's patent). In conclusion, MICA is a potential therapeutic target. It is of great value to develop a MICA-targeted monoclonal antibody medicament.

In a first aspect of the present disclosure, the present disclosure provides an antibody or antigen-binding fragment thereof. According to embodiments of the present disclosure, the antibody or antigen-binding fragment thereof includes: a heavy chain variable region CDR1 sequence, a heavy chain variable region CDR2 sequence, and a heavy chain variable region CDR3 sequence as set forth in SEQ ID NO: 1, SEQ ID NO: 2, and SEQ ID NO: 3, respectively, or as set forth in amino acid sequences having at least 80% identity to SEQ ID NO: 1, SEQ ID NO: 2, and SEQ ID NO: 3, respectively; and a light chain variable region CDR1 sequence, a light chain variable region CDR2 sequence, and a light chain variable region CDR3 sequence as set forth in SEQ ID NO: 4, SEQ ID NO: 5, and SEQ ID NO: 6, respectively, or as set forth in amino acid sequences having at least 80% identity to SEQ ID NO: 4, SEQ ID NO: 5, and SEQ ID NO: 6, respectively.

According to the embodiments of the present disclosure, the antibody or antigen-binding fragment thereof can bind to MICA with a high affinity.

According to the embodiments of the present disclosure, the antibody or antigen-binding fragment thereof may further include at least one of additional technical features as described below.

According to the embodiments of the present disclosure, the antibody or antigen-binding fragment thereof includes: the heavy chain variable region CDR1 sequence as set forth in SEQ ID NO: 1; the heavy chain variable region CDR2 sequence as set forth in SEQ ID NO: 2; the heavy chain variable region CDR3 sequence as set forth in SEQ ID NO: 3; the light chain variable region CDR1 sequence as set forth in SEQ ID NO: 4; the light chain variable region CDR2 sequence as set forth in SEQ ID NO: 5; and the light chain variable region CDR3 sequence as set forth in SEQ ID NO: 6.

According to the embodiments of the present disclosure, the antibody or antigen-binding fragment thereof is capable of specifically recognizing.

According to the embodiments of the present disclosure, the antibody includes a heavy chain variable region and a light chain variable region, an amino acid sequence of the heavy chain variable region being as set forth in SEQ ID NO: 7, and an amino acid sequence of the light chain variable region being as set forth in SEQ ID NO: 8.

According to the embodiments of the present disclosure, the antibody is a humanized antibody.

According to the embodiments of the present disclosure, the antibody or antigen-binding fragment thereof includes a heavy chain frame region sequence and a light chain frame region sequence, wherein at least one part of at least one of the heavy chain frame region sequence and the light chain frame region sequence is derived from at least one of a murine antibody, a humanized antibody, a primate-derived antibody, or a mutant thereof.

According to the embodiments of the present disclosure, the antibody or antigen-binding fragment thereof includes at least one of a heavy chain constant region and a light chain constant region, wherein at least one part of the at least one of the heavy chain constant region and the light chain constant region is derived from at least one of a humanized antibody, a primate-derived antibody, a murine antibody, or a mutant thereof.

According to the embodiments of the present disclosure, the light chain constant region and the heavy chain constant region are both derived from a murine IgG1 antibody, a murine IgG2a antibody, or a mutant thereof; or a human IgG1 antibody, a human IgG2 antibody, a human IgG3 antibody, a human IgG4 antibody, or a mutant thereof.

According to the embodiments of the present disclosure, the antibody or antigen-binding fragment thereof is at least one of a single chain antibody, a multimeric antibody, a CDR transplant antibody, a Fab antibody, or an Fv antibody.

In a second aspect of the present disclosure, the present disclosure provides a nucleic acid molecule. According to the embodiments of the present disclosure, the nucleic acid molecule encodes the antibody or antigen-binding fragment thereof according to the first aspect.

It should be noted that, those skilled in the art should understand that, nucleic acids mentioned in the description and claims of the present disclosure actually include any one or two of complementary double strands. For convenience, in the present description and the claims, although only one strand is given in most cases, the other strand complementary to the one strand is also disclosed actually. In addition, nucleotide sequences in the present disclosure include a DNA form or an RNA form. When one form is disclosed, it means that the other form is also disclosed.

In a third aspect of the present disclosure, the present disclosure provides an expression vector. According to the embodiments of the present disclosure, the expression vector carries the nucleic acid molecule according to the second aspect. The expression vector may include an optional control sequence. The control sequence is operably linked with said nucleic acid molecule. The control sequence refers to one or more control sequences that direct the expression of said nucleic acid molecule in the host. The expression vector provided in the embodiments of the present disclosure can efficiently and highly express said antibody or antigen-binding fragment thereof in suitable host cells.

The expression vector provided in the embodiments of the present disclosure can efficiently express the antibody or antigen-binding fragment thereof in suitable receptor cells. The antibody or the antigen-binding fragment thereof, as designed in the present disclosure, has higher specificity and higher safety.

In a fourth aspect of the present disclosure, the present disclosure provides a method for preparing the antibody or antigen-binding fragment thereof. According to the embodiments of the present disclosure, the method includes: introducing the expression vector according to the third aspect into cells; and culturing the cells under conditions suitable for protein expression and secretion, to obtain the antibody or antigen-binding fragment thereof.

The Applicant found that, according to the embodiments of the present disclosure, the antibody or antigen-binding fragment thereof can be efficiently prepared with high purity and simple operating steps, and the cost is low.

According to some specific implementations of the present disclosure, the cells are not specially limited and can be prokaryotic cells or eukaryotic cells.

According to some specific implementations of the present disclosure, the cells are eukaryotic cells.

According to some specific implementations of the present disclosure, the eukaryotic cells are mammalian cells. According to some specific embodiments of the present disclosure, when the cells are the eukaryotic cells such as the mammalian cells, a recombinant antibody has higher expression efficiency.

In a fifth aspect of the present disclosure, the present disclosure provides a recombinant cell. According to the embodiments of the present disclosure, the recombinant cell expresses the antibody or antigen-binding fragment thereof according to the first aspect, or carries the nucleic acid molecule according to the second aspect or the expression vector according to the third aspect. The recombinant cell is obtained by transfection or transformation of the expression vector. According to some specific implementations of the present disclosure, the recombinant cell can efficiently and highly express the antibody or antigen-binding fragment thereof under suitable conditions.

According to some specific embodiments of the present disclosure, the recombinant cell can efficiently and highly express the antibody or antigen-binding fragment thereof under suitable conditions. Said antibody or antigen-binding fragment thereof has higher specificity, longer half-life period and higher efficacy, and an antibody medicament can be delivered to target cells at a lower dose, thereby effectively treating or preventing a MICA-mediated disease. Moreover, the recombinant cell has lower toxic and side effects and higher safety.

It should be noted that, the recombinant cell according to the present disclosure is not specially limited, and it may be a prokaryotic cell, a eukaryotic cell, or a phage. The prokaryotic cell may be *Escherichia coli*, *Bacillus subtilis, Streptomyces,* or *Proteus mirabilis.* The eukaryotic cell includes: fungi such as *Pichia pastoris, Saccharomyces cerevisiae, Schizosaccharomyces,* and *Trichoderma;* insect cells such as grass armyworms; plant cells such as tobacco; and mammalian cells such as BHK cells, CHO cells, COS cells, and myeloma cells. In some embodiments, the recombinant cells according to the present disclosure are preferably the mammalian cells, including BHK cells, CHO cells, NSO cells, or COS cells and do not include animal germ cells, fertilized egg or embryonic stem cells.

It should be noted that, the "suitable conditions" in the description of the present disclosure refer to conditions suitable for the expression of the antibody or antigen-binding fragment thereof according to the present disclosure. Those skilled in the art can easily conceive that, the conditions suitable for the expression of the antibody or antigen-binding fragment thereof include, but not limited to, a suitable transformation or transfection mode, a suitable transformation or transfection condition, a healthy host cell state, a suitable density of host cells, a suitable culture environment, and suitable culture period. The "suitable conditions" are not specially limited. The most suitable conditions for the expression of the antibody or antigen-binding fragment thereof may be optimized by those skilled in the art based on specific laboratory environment.

In a sixth aspect of the present disclosure, the present disclosure provides a composition. According to the embodiments of the present disclosure, the composition includes the antibody or antigen-binding fragment thereof according to the first aspect, the nucleic acid molecule according to the second aspect, the expression vector according to the third aspect, or the recombinant cell according to the fifth aspect.

As mentioned above, the antibody or antigen-binding fragment thereof in some specific implementations of the present disclosure can effectively bind to the huma MICA protein and can thus effectively inhibit proliferation of tumor cells. Therefore, the composition including the above substances can also effectively bind to the huma MICA protein and has excellent effects of preventing and/or treating the MICA-mediated disease. The type of the composition is not specially limited. The composition may be a food composition or a pharmaceutical composition.

The composition according to the present disclosure can be combined with each other, or it can be administered in combination with one or more other therapeutic compounds, for example, in combination with a chemotherapeutic agent. Therefore, the composition may further include a chemotherapeutic agent. The antibody or the antigen-binding fragment thereof, the nucleic acid molecule, the expression vector, or the recombinant cell according to the present disclosure may be in combination with a second therapeutic agent. Exemplary agents of the second therapeutic agent include, but not limited to: other reagents that inhibit MICA activity, including other antibodies or antigen-binding fragments thereof, peptide inhibitors, small molecule antagonists, etc.; and/or agents interfering with MICA upstream or downstream signal transduction.

In some implementations, the composition includes combinations separated temporally or spatially, as long as the purpose of the present disclosure can be achieved based on the cooperation of the combinations. For example, components in the composition may be applied to a subject as a whole or separately. When the components contained in the composition are separately applied to the subject, the respective components may be simultaneously or sequentially applied to the subject.

In general, the antibody or antigen-binding fragment thereof is administered at an effective dose, which is a dose sufficient to reach an expected treatment and/or prevention effect. For example, the effective dose is a dose capable of preventing or relieving symptoms related to a to-be-treated disease, which refers to, for example, a MICA-associated disease. The effective dose of the composition administered to the subject depends on the type and severity of the disease as well as characteristics of individuals, for example, general health state, age, gender, weight, and tolerance to the medicament. The effective dose further depends on the severity and the type of the diseases. An appropriate dose can be determined by those skilled in the art according to these factors.

According to the embodiments of the present disclosure, the composition according to the present disclosure has the same therapeutic effects as the antibody or antigen-binding fragment thereof, the nucleic acid molecule, the expression vector or the recombinant cell when entering an organism.

In a seventh aspect of the present disclosure, the present disclosure provides use of the antibody or antigen-binding fragment thereof according to the first aspect, the nucleic acid molecule according to the second aspect, the expression vector according to the third aspect, the recombinant cell according to the fifth aspect, or the composition according to the sixth aspect in preparation of a medicament. According to the embodiments of the present disclosure, the medicament is used for preventing and/or treating a MICA-mediated disease. As mentioned above, the antibody or antigen-binding fragment thereof in some specific implementations of the present disclosure can effectively bind to a huma MICA protein. Therefore, the medicament including the antibody or antigen-binding fragment thereof in an effective dose or a series of substances thereof can also effectively bind to the huma MICA protein, and thus can have an excellent effect of preventing and/or treating the MICA-mediated disease, such as cancers.

According to the embodiments of the present disclosure, the MICA-mediated disease is a cancer.

According to the embodiments of the present disclosure, the cancer is at least one of lung cancer, liver cancer, ovarian cancer, cervical cancer, skin cancer, bladder cancer, colon cancer, breast cancer, glioma, renal cancer, gastric cancer, esophagus cancer, oral squamous cell carcinoma, and head-and-neck cancer.

In an eighth aspect of the present disclosure, the present disclosure provides a medicament. According to the embodiments of the present disclosure, the medicament includes the antibody or antigen-binding fragment thereof according to the first aspect, the nucleic acid molecule according to the second aspect, the expression vector according to the third aspect, the recombinant cell according to the fifth aspect, or the composition according to the sixth aspect. The medicament is used for preventing and/or treating a MICA-mediated disease.

According to the embodiments of the present disclosure, the MICA-mediated disease is a cancer.

According to the embodiments of the present disclosure, the cancer is at least one of lung cancer, liver cancer, ovarian cancer, cervical cancer, skin cancer, bladder cancer, colon cancer, breast cancer, glioma, renal cancer, gastric cancer, esophagus cancer, oral squamous cell carcinoma, and head-and-neck cancer.

According to the embodiments of the present disclosure, the medicament may further include a pharmaceutically acceptable carrier.

The effective dose of the antibody or antigen-binding fragment thereof according to the present disclosure may vary with a mode of administration and severity of the to-be-treated disease. Preferably, the effective dose may be determined by those skilled in the art based on various factors (such as though a clinical test). The factors include, but not limited to: pharmacokinetic parameters of active ingredients, for example, bioavailability, metabolism, and half-life period; severity of the to-be-treated diseases of patients; weight of the patients; immune states of the patients; and routes of administration. For example, based on the urgent requirements of treatment states, separated doses may be administrated several times per day, or the dose can be reduced proportionally.

In a ninth aspect of the present disclosure, the present disclosure provides use of the antibody or antigen-binding fragment thereof according to the first aspect, the nucleic acid molecule according to the second aspect, the expression vector according to the third aspect, or the recombinant cell according to the fifth aspect in the preparation of a kit. According to the embodiments of the present disclosure, the kit is used for detecting MICA. As mentioned above, the antibody or antigen-binding fragment thereof in some specific implementations of the present disclosure can effectively bind to a huma MICA protein. Therefore, the antibody or antigen-binding fragment thereof may be used for preparing a kit for detecting the MICA protein. A qualitative or quantitative detection can be effectively performed on the huma MICA protein with the kit.

According to the embodiments of the present disclosure, the kit can efficiently and accurately detect MICA, thereby saving time and exploration cost for clinical treatment.

In a tenth aspect of the present disclosure, the present disclosure provides a kit. According to the embodiments of the present disclosure, the kit includes the antibody or antigen-binding fragment thereof according to the first aspect, the nucleic acid molecule according to the second aspect, the expression vector according to the third aspect, or the recombinant cell according to the fifth aspect.

As mentioned above, the antibody or antigen-binding fragment thereof in some specific implementations of the present disclosure can effectively bind to the huma MICA protein. Therefore, with the kit including the antibody or antigen-binding fragment thereof, a qualitative or quantitative detection can be effectively performed on the huma MICA protein. The kit provided by the present disclosure, for instance, may be a kit involving a specific binding of huma MICA and the antibody, for example, a kit for immunoblotting, or a kit for immunoprecipitation. Such a kit may include any or several of antagonists, anti-MICA antibodies, or medicament reference materials; protein purification columns; immunoglobulin affinity purification buffering agents; cell determination diluents; manual or literatures. The anti-MICA antibodies may be used for diagnostic tests of different types, for example, for detecting existence of various diseases or medicaments, toxins or other proteins *in vitro* or *in vivo;* for instance, for diagnosing related diseases by detecting serum or blood of subjects, and for conducting scientific researches. The huma MICA protein in a to-be-detected sample is detected using the kit. The related diseases may include MICA related diseases, such as cancers. Certainly, the antibody or antigen-binding fragment thereof provided in the present disclosure may also be used for radioimmunoassay and radioimmunotherapy of the above diseases. With respect to the above application scenarios, the binding molecule is also applicable, which is not described in detail herein.

According to some specific embodiments of the present disclosure, the kit may further include, for example, a coating solution, which is conventional for MICA detection.

In an eleventh aspect of the present disclosure, the present disclosure provides a method for treating or preventing a related disease caused by MICA. According to the embodiments of the present disclosure, the method includes a step of administrating to a subject at least one of: 1) the antibody or antigen-binding fragment thereof according to the first aspect; 2) the nucleic acid molecule according to the second aspect; 3) the expression vector according to the third aspect; 4) the recombinant cell according to the fifth aspect; 5) the composition according to the sixth aspect; and 6) the medicament according to the eighth aspect. As mentioned above, the antibody or antigen-binding fragment thereof can bind to the huma MICA protein, thereby effectively treating or preventing the related disease caused by MICA, such as cancer. Therefore, the method according to the embodiments of the present disclosure can effectively treat or prevent the related disease caused by MICA.

According to the embodiments of the present disclosure, the cancers include at least one of lung cancer, liver cancer, ovarian cancer, cervical cancer, skin cancer, bladder cancer, colon cancer, breast cancer, glioma, renal cancer, gastric cancer, esophagus cancer, oral squamous cell carcinoma, and head-and-neck cancer.

In a twelfth aspect of the present disclosure, the present disclosure provides a method for diagnosing a MICA-mediated disease. According to the embodiments of the present disclosure, the method includes a step of detecting MICA in a to-be-detected sample by using at least one of: 1) the antibody or antigen-binding fragment thereof according to the first aspect; 2) the nucleic acid molecule according to the second aspect; 3) the expression vector according to the third aspect; and 4) the recombinant cell according to the fifth aspect. A content of MICA in the to-be-detected sample is determined based on a detection result of MICA. The antibody or the antigen-binding fragment thereof provided by the present disclosure, or antibodies or antigen-binding fragments thereof expressed by the nucleic acid molecule, the expression vector and the recombinant cell according to the present disclosure may effectively bind to the huma MICA protein. Therefore, the method according to the present disclosure can effectively detect the content of MICA in the to-be-detected sample derived from a subject, and thus the method can further effectively diagnose the related diseases caused by MICA.

According to the embodiments of the present disclosure, the method for disease diagnosing may further include at least one of additional technical features as below.

According to the embodiments of the present disclosure, an indication that the to-be-detected sample is derived from a patient with the related disease caused by MICA is that the content of MICA in the to-be-detected sample is not lower than the lowest standard of suffering from such a disease. A value of the lowest standard may be determined by performing difference comparative analysis and validation on the contents of MICA in to-be-detected samples derived from a large number of individuals suffering from the MICA-mediated disease and a large number of healthy individuals.

According to the embodiments of the present disclosure, the to-be-detected sample includes at least one of blood, saliva, sweat, tissues, cells, blood, serum, plasma, excrements, and urine.

According to the embodiments of the present disclosure, the related diseases caused by MICA include cancers.

According to the embodiments of the present disclosure, the cancer includes at least one of lung cancer, liver cancer, ovarian cancer, cervical cancer, skin cancer, bladder cancer, colon cancer, breast cancer, glioma, renal cancer, gastric cancer, esophagus cancer, oral squamous cell carcinoma, and head-and-neck cancer.

In a thirteenth aspect of the present disclosure, the present disclosure provides a method for staging a related disease caused by MICA. According to the embodiments of the present disclosure, the method includes a step of detecting MICA in a to-be-detected sample by using at least one: 1) the antibody or antigen-binding fragment thereof according to the first aspect; 2) the nucleic acid molecule according to the second aspect; 3) the expression vector according to the third aspect; and 4) the recombinant cell according to the fifth aspect. A content of MICA in the to-be-detected sample is determined based on a detection result of MICA. The antibody or the antigen-binding fragment thereof provided by the present disclosure, or antibodies or antigen-binding fragments thereof expressed by the nucleic acid molecule, the expression vector and the recombinant cell according to the present disclosure can effectively bind to the huma MICA protein. Therefore, the method according to the present disclosure can effectively detect the content of MICA in the to-be-detected sample from a subject, and thus the method can further evaluate the stage of the related disease caused by MICA based on the content of MICA.

According to the embodiments of the present disclosure, the method for staging the diseases may further include at least one of additional technical features as below.

According to the embodiments of the present disclosure, an indication that the to-be-detected sample is derived from a patient with a stage IV tumor is in that the content of MICA in the to-be-detected sample is not lower than a standard level of patients suffering from the stage IV tumor; an indication that the to-be-detected sample is derived from a patient with the stage III tumor is in that the content of MICA in the to-be-detected sample is between a standard level of patients suffering from a stage IV tumor and a standard level of patients suffering from a stage III tumor; an indication that the to-be-detected sample is derived from a patient with a stage II tumor is in that the content of MICA in the to-be-detected sample is between a standard level of patients suffering from a stage III tumor and a standard level of patients suffering from a stage II tumor; and an indication that the to-be-detected sample is derived from a patient with a stage I tumor is in that that the content of MICA in the to-be-detected sample is between a standard level of patients suffering from a stage I tumor and a standard level of patients suffering from a stage II tumor. Those skilled in the art can understand that, during patients suffering from the tumor stages I, II, III and IV, the MICA level varies with different tumor types; and the stages of the tumor can be determined by comparing the content of MICA in the to-be-detected sample with the standard level of MICA at a corresponding tumor stage, or by comparing the content of MICA in the to-be-detected sample with a content of MICA in samples from individuals or groups at a known disease stage. Values of the standard levels at the tumor stages I, II, III and IV may be determined by performing difference comparative analysis and validation on the contents of MICA in to-be-detected samples of a large number of individuals suffering from the related diseases caused by MICA and a large number of healthy individuals.

According to the embodiments of the present disclosure, the to-be-detected sample includes at least one of blood, saliva, sweat, tissues, cells, blood, serum, plasma, excrements, and urine.

According to the embodiments of the present disclosure, the related diseases caused by MICA include cancers.

According to the embodiments of the present disclosure, the cancers include at least one of lung cancer, liver cancer, ovarian cancer, cervical cancer, skin cancer, bladder cancer, colon cancer, breast cancer, glioma, renal cancer, gastric cancer, esophagus cancer, oral squamous cell carcinoma, and head-and-neck cancer.

In a fourteenth aspect of the present disclosure, the present disclosure provides a method for evaluating prognosis of a related disease caused by MICA. According to the embodiments of the present disclosure, the method includes a step of detecting MICA in a to-be-detected sample by using at least one of: 1) the antibody or antigen-binding fragment thereof according to the first aspect; 2) the nucleic acid molecule according to the second aspect; 3) the expression vector according to the third aspect; and 4) the recombinant cell according to the fifth aspect. A content of MICA in the to-be-detected sample is determined based on a detection result of MICA. As mentioned above, the content of MICA has significant influence on the cancers. After the individuals suffering from the related diseases are treated, the prognosis of the diseases may be effectively evaluated by monitoring the contents of MICA in tissues or excrements (peripheral blood and urine) of the individuals, for instance, in a manner of comparing the contents of MICA in the body of the subjects before and after treatment, or comparing the contents of MICA in the body of the subjects after treatment with the MICA level of normal individuals or diseased individuals. The antibody or the antigen-binding fragment thereof provided by the present disclosure, or antibodies or antigen-binding fragments thereof expressed by the nucleic acid molecule, the expression vector and the recombinant cell according to the present disclosure can effectively bind to huma MICA. Therefore, the method according to the present disclosure can effectively detect the content of MICA in the to-be-detected sample of the tested individuals, and thus the method can further evaluate the prognosis of the related diseases caused by MICA based on the content of MICA.

According to the embodiments of the present disclosure, the method for evaluating the disease prognosis may further include at least one of additional technical features as below.

According to the embodiments of the present disclosure, the to-be-detected sample is derived from patients suffering from related disease caused by MICA before and after treatment.

According to the embodiments of the present disclosure, the to-be-detected sample includes at least one of blood, saliva, sweat, tissues, cells, blood, serum, plasma, excrements, and urine.

According to the embodiments of the present disclosure, a prognosis effect of the related disease caused by MICA is determined based on the content of MICA in the to-be-detected sample of the patient suffering from the related disease caused by MICA before and after treatment.

According to the embodiments of the present disclosure, the related diseases caused by MICA include cancers.

According to the embodiments of the present disclosure, the cancers include at least one of lung cancer, liver cancer, ovarian cancer, cervical cancer, skin cancer, bladder cancer, colon cancer, breast cancer, glioma, renal cancer, gastric cancer, esophagus cancer, oral squamous cell carcinoma, and head-and-neck cancer.

In a fifteenth aspect of the present disclosure, the present disclosure provides use of the antibody or antigen-binding fragment thereof according to the first aspect, the nucleic acid molecule according to the second aspect, the expression vector according to the third aspect, the recombinant cell according to the fifth aspect, the composition according to the sixth aspect, or the medicament according to the eighth aspect in the treatment or prevention of a related disease caused by MICA. As mentioned above, the antibody or antigen-binding fragment thereof can effectively bind to the huma MICA, thereby effectively treating or preventing the related disease caused by MICA.

According to the embodiments of the present disclosure, the use may further include at least one of additional technical features as below.

According to the embodiments of the present disclosure, the related diseases caused by MICA include cancers.

According to the embodiments of the present disclosure, the cancers include at least one of lung cancer, liver cancer, ovarian cancer, cervical cancer, skin cancer, bladder cancer, colon cancer, breast cancer, glioma, renal cancer, gastric cancer, esophagus cancer, oral squamous cell carcinoma, and head-and-neck cancer.

In a sixteenth aspect of the present disclosure, the present disclosure provides use of the antibody or antigen-binding fragment thereof according to the first aspect, the nucleic acid molecule according to the second aspect, the expression vector according to the third aspect, or the recombinant cell according to the fifth aspect in the diagnosis of a MICA-mediated disease, staging of the MICA-mediated disease or evaluation of prognosis of the MICA-mediated disease. As mentioned above, the antibody or antigen-binding fragment thereof provided by the present disclosure, or antibodies or antigen-binding fragments thereof expressed by the nucleic acid molecule, the expression vector and the recombinant cell may effectively bind to huma MICA. Therefore, the method in the present disclosure can effectively detect the content of the MICA in the to-be-detected samples of the tested individuals, and further perform effective diagnosis, staging and prognosis evaluation on the MICA-mediated diseases.

According to the embodiments of the present disclosure, the use may further include at least one of additional technical features as below.

According to the embodiments of the present disclosure, the related diseases caused by MICA include cancers.

According to the embodiments of the present disclosure, the cancers include at least one of lung cancer, liver cancer, ovarian cancer, cervical cancer, skin cancer, bladder cancer, colon cancer, breast cancer, glioma, renal cancer, gastric cancer, esophagus cancer, oral squamous cell carcinoma, and head-and-neck cancer.

The "subject" or "individual" involved in the present disclosure refer to a mammal, e.g., primate and/or rodent, particularly human, monkey, or mouse.

### Beneficial effects of the present disclosure

1) Compared with a maternal antibody h5A1 and a homogeneous antibody CLN-619 (Cullinan, at a clinical stage I), the antibody h5A1002 with affinity maturation obtained in the present disclosure has higher binding activity.
2) Compared with the maternal antibody h5A1 and the homogeneous antibody CLN-619 (Cullinan, at a clinical stage I), the antibody h5A1002 with affinity maturation obtained in the present disclosure can more intensively promote an activity of peripheral blood mononuclear cells (PBMC) for killing tumors.
3) Compared with the homogeneous antibody CLN-619 (Cullinan, at a clinical stage I), the antibody h5A1002 with affinity maturation obtained in the present disclosure has lower endocytic activity, and has higher drugability when using as a monoclonal antibody or a double antibody.

### BRIEF DESCRIPTION OF THE DRAWINGS

The above and/or additional aspects and advantages of the present disclosure will become obvious and be easily understood from descriptions of embodiments in combination with drawings below.

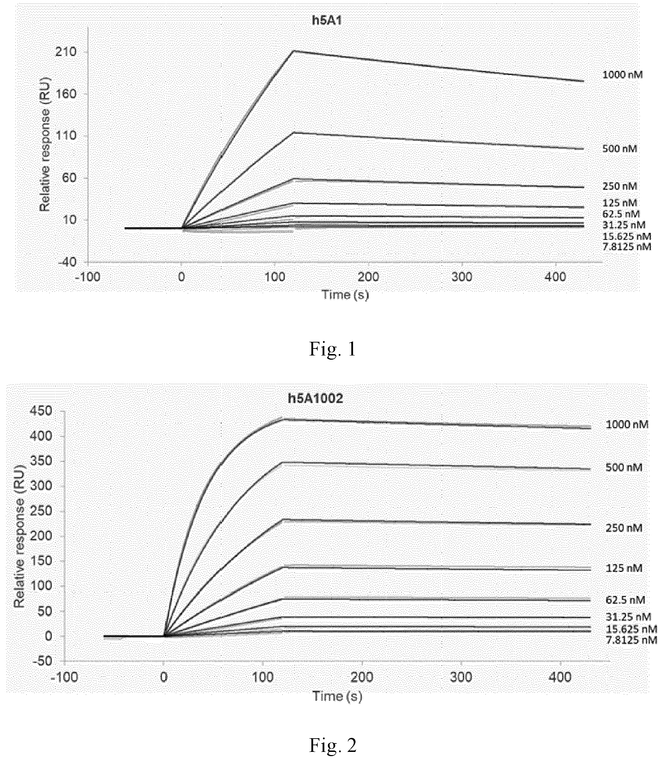

Fig. 1 is a SPR result diagram of determination of an affinity of a maternal humanized antibody h5A1 according to embodiments of the present disclosure to MICA protein;
Fig. 2 is a SPR result diagram of determination of an affinity of an antibody h5A1002 with affinity maturation according to embodiments of the present disclosure to MICA protein;
Fig. 3 is an ELISA result diagram of binding of a maternal antibody h5A1 and antibody h5A1002 with affinity maturation according to embodiments of the present disclosure to MICA*002-Fc protein;
Fig. 4 is an ELISA result diagram of binding of a maternal antibody h5A1 and antibody h5A1002 with affinity maturation according to embodiments of the present disclosure to MICA*005-Fc protein;
Fig. 5 is an ELISA result diagram of binding of a maternal antibody h5A1 and antibody h5A1002 with affinity maturation according to embodiments of the present disclosure to MICA*008-Fc protein;
Fig. 6 is an ELISA result diagram of binding of a maternal antibody h5A1 and antibody h5A1002 with affinity maturation according to embodiments of the present disclosure to MICA*004-Fc protein;
Fig. 7 is an ELISA result diagram of binding of a maternal antibody h5A1 and antibody h5A1002 with affinity maturation according to embodiments of the present disclosure to MICB*001-Fc protein;
Fig. 8 is an ELISA result diagram of binding of a maternal antibody h5A1 and antibody h5A1002 with affinity maturation according to embodiments of the present disclosure to MICB*005-Fc protein;
Fig. 9 is a flow cytometry result diagram of binding of a maternal antibody h5A1 and antibody h5A1002 with affinity maturation according to embodiments of the present disclosure to human malignant melanoma A-375 cells;
Fig. 10 is a flow cytometry result diagram of binding of a maternal antibody h5A1 and antibody h5A1002 with affinity maturation according to embodiments of the present disclosure to human colorectal cancer HCT-15 cells;
Fig. 11 is an ELISA result diagram of binding of an antibody h5A1002 with affinity maturation according to embodiments of the present disclosure, homogeneous antibodies CLN-619 (Cullinan) and 1D5V11 (Genentech) to MICA*002-Fc protein;
Fig. 12 is an ELISA result diagram of binding of an antibody h5A1002 with affinity maturation according to embodiments of the present disclosure, homogeneous antibodies CLN-619 (Cullinan) and 1D5V11 (Genentech) to MICA*005-Fc protein;
Fig. 13 is an ELISA result diagram of binding of an antibody h5A1002 with affinity maturation according to embodiments of the present disclosure, homogeneous antibodies CLN-619 (Cullinan) and 1D5V11 (Genentech) to MICA*008-Fc protein;
Fig. 14 is an ELISA result diagram of binding of an antibody h5A1002 with affinity maturation according to embodiments of the present disclosure, homogeneous antibodies CLN-619 (Cullinan) and 1D5V11 (Genentech) to MICA*004-Fc protein;
Fig. 15 is an ELISA result diagram of binding of an antibody h5A1002 with affinity maturation according to embodiments of the present disclosure, homogeneous antibodies CLN-619 (Cullinan) and 1D5V11 (Genentech) to MICB*001-Fc protein;
Fig. 16 is an ELISA result diagram of binding of an antibody h5A1002 with affinity maturation according to embodiments of the present disclosure, homogeneous antibodies CLN-619 (Cullinan) and 1D5V11 (Genentech) to MICB*005-Fc protein;
Fig. 17 is a flow cytometry result diagram of binding of an antibody h5A1002 with affinity maturation according to embodiments of the present disclosure, homogeneous antibodies CLN-619 (Cullinan) and 1D5V11 (Genentech) to human malignant melanoma A-375 cells;
Fig. 18 is a flow cytometry result diagram of binding of an antibody h5A1002 with affinity maturation according to embodiments of the present disclosure, homogeneous antibodies CLN-619 (Cullinan) and 1D5V11 (Genentech) to human non-small cell lung cancer A549 cells;
Fig. 19 is a flow cytometry result diagram of binding of an antibody h5A1002 with affinity maturation according to embodiments of the present disclosure, homogeneous antibodies CLN-619 (Cullinan) and 1D5V11 (Genentech) to human colorectal cancer HCT-15 cells;
Fig. 20 is a flow cytometry result diagram of binding of an antibody h5A1002 with affinity maturation according to embodiments of the present disclosure, homogeneous antibodies CLN-619 (Cullinan) and 1D5V11 (Genentech) to human breast cancer MDA-MB-231 cells;
Fig. 21 is a flow cytometry result diagram of binding of an antibody h5A1002 with affinity maturation according to embodiments of the present disclosure, homogeneous antibodies CLN-619 (Cullinan) and 1D5V11 (Genentech) to human colorectal cancer HCT-15 cells in competitive with an antibody h5A1002-Biotin;
Fig. 22 is a flow cytometry result diagram of endocytosis of an antibody h5A1002 with affinity maturation according to embodiments of the present disclosure and homogeneous antibodies CLN-619 (Cullinan) and 1D5V11 (Genentech) by human breast cancer MDA-MB-231 cells;
Fig. 23 is a result diagram of an effect of an antibody h5A1002 with affinity maturation according to embodiments of the present disclosure in promoting PBMCs for killing human malignant melanoma A-375 cells;
Fig. 24 is a result diagram of an effect of an antibody h5A1002 with affinity maturation according to embodiments of the present disclosure and homogeneous antibodies CLN-619 (Cullinan) and 1D5V11 (Genentech) in promoting PBMCs for killing human colorectal cancer HCT-15 cells; and
Fig. 25 is a result diagram of an effect of an antibody h5A1002 with affinity maturation according to embodiments of the present disclosure and homogeneous antibodies CLN-619 (Cullinan) and 1D5V11 (Genentech) in promoting PBMCs for killing human malignant melanoma A-375 cells.

### DETAILED DESCRIPTION

Embodiments of the present disclosure will be described in detail below. The embodiments described below are illustrative, are merely used for explaining the present disclosure, and cannot be understood as a limitation to the present disclosure.

It should be noted that, terms "first" and "second" are merely used for a descriptive purpose, but cannot be understood as an indication or implication of relative importance or an implied specification of the quantity of indicated technical features. Thus, features defined with the "first" and "second" may explicitly or implicitly include one or more features. Further, in the descriptions of the present disclosure, unless otherwise noted, the meaning of "more" refers to two or more than two.

In the present disclosure, the term "include" or "comprise" is an open inclusion expression, i.e., including the contents specified in the present disclosure, but not excluding other contents.

In the present disclosure, terms "optionally", and "optional" generally mean that subsequent events or statuses may occur but not necessarily occur; and the descriptions include conditions under which the events or statuses occur, and conditions under which the events or statuses do not occur.

In order to facilitate understanding of the present disclosure, some technical and scientific terms are specifically defined below. Unless obviously and clearly defined in other parts of the present document, all other technical and scientific terms used in the present disclosure have meanings commonly understood by those skilled in the art in the present disclosure. An abbreviation of amino acid residues is a standard 3-letter and/or 1-letter code that refers to one of the 20 commonly used L-amine acids in the art.

An antibody or antigen-binding fragment thereof according to the present disclosure is generally prepared by a biosynthesis method. According to nucleotide sequences in the present disclosure, coded nucleic acids according to the present disclosure may be conveniently prepared by those skilled in the art using various known methods. For example, these methods include, but not limited to: PCR, DNA artificial synthesis, etc. A specific method may refer to J. Sambrook, Molecular Cloning: Laboratory Manual*.* As an implementation of the present disclosure, coded nucleic acid sequences may be constructed by a method for synthesizing nucleotide sequences by stages and conducting overlap extension PCR. The antibody or the antigen fragment is numbered and defined by a Kabat numbering system.

In the present disclosure, the "monoclonal antibody" refers to an antibody with a single antigen-binding site.

In the present disclosure, the "polyclonal antibody" refers to an antibody with two or more different antigen-binding sites.

In the present disclosure, the term "mutant" or "variant" may refer to a molecule obtained by performing mutation of one or more nucleotides or amino acids on any natural or engineered molecule.

The term "complementary determining region" or "CDR" or "CDR sequence" refers to an amino acid sequence for antigen-binding in the antibody, and for instance, it generally includes: amino acid residues nearby 23-34(L1), 50-56(L2) and 89-97(L3) in a light chain variable region, and amino acid residues nearby 31-35B(H1), 50-65(H2) and 95-102(H3) in a heavy chain variable region (Kabat et al., Sequences of Proteins of Immunological Interest, 5th Ed. Public Health Service, National Institutes of Health, Bethesda, MD.(1991)); and/or amino acid residues from "hypervariable loop" (for instance, amino acid residues nearby 26-32(LI), 50-52(L2) and 91-96(L3) in the light chain variable region, and nearby 26-32(H1), 53-55(H2) and 96-101(H3) in the heavy chain variable region) (Chothia and Lesk J. Mol. Biol. 196: 901-917(1987)).

The "operable link" in the present disclosure means that an exogenous gene is linked to a vector, enabling control components in the vector, such as a transcriptional control sequence and a translational control sequence, to exert an expected effect of regulating transcription and translation of the exogenous gene. When the nucleic acid molecule is linked to the vector, the nucleic acid molecule may be directly or indirectly connected with the control components on the vector, as long as these control components can control translation and expression of the nucleic acid molecule. These control components may be directly derived from the vector, and they may also be exogenous, that is, not from the vector. It may be understood by those skilled in the art that, the nucleic acid molecule used for coding the antibody or the antigen-binding fragment may be respectively and independently inserted into different vectors, and is commonly inserted onto the same vector. Common vectors, for instance, may be plasmids, phage, etc., such as Plasmid-X.

In the present disclosure, when terms "identity", "homology" or "similarity" are used for describing amino acid sequences or nucleic acid sequences relative to reference sequences, a percentage of the same amino acid or nucleotide between two amino acid sequences or nucleic acid sequences is determined by a conventional method, for instance, referring to Ausubel, et al., Current Protocols in Molecular Biology, Chapter 19 (Greene Publishing and Wiley-Interscience, 1995, New York); and ALIGN program (Dayhoff (1978), Atlas of Protein Sequence and Structure 5: Suppl.3 (National Biomedical Research Foundation, Washington, D.C.). Various algorithms are applicable to sequence alignment and sequence identity determination and include: a homology alignment algorithm of Needleman et al. (1970) J.Mol.Biol.48: 443; a local homology algorithm of Smith et al. (1981) Adv.Appl.Math.2: 482; a similarity search method of Pearson et al. (1988) Proc.Natl.Acad.Sci.85: 2444; a Smith-Waterman algorithm (Meth.Mol.Biol.70: 173-187(1997)); and BLASTP, BLASTN and BLASTX algorithms (referring to Altschul et al. (1990) J.Mol.Biol.215: 403-410). Computer programs employing these algorithms are also available, and they include, but not limited to: ALIGN or Megalign (DNASTAR) software, or WU-BLAST-2 (Altschul et al., Meth. Enzym., 266: 460-480(1996)); or GAP, BESTFIT, BLAST Altschul etc., FASTA and TFASTA, available in Genetics Computing Group (GCG) packet, Version 8, Madison, Wisconsin, USA; and CLUSTAL in a PC/Gene program provided by Intelligenetics, Mountain View, California.

Without substantially affecting antibody activity (remaining at least 95% of activity), substitution, addition and/or deletion of one or more amino acids (such as, 1, 2, 3, 4, 5, 6, 7, 8, 9, or 10 or more) may be conducted by those skilled in the art on the sequences in the present disclosure, to obtain variants of sequences of the antibody or functional fragments thereof. These variants may be regarded as being included in the protection scope of the present disclosure. For example, amino acids with similar properties are substituted in a variable region. The variant sequences in the present disclosure may have consistency (or homology) of at least 80%, 81%, 82%, 83%, 84%, 85%, 86%, 87%, 88%, 89%, 90%, 91%, 92%, 93%, 94%, 95%, 96%, 97%, 98% or 99% to the reference sequences. The sequence consistency in the present disclosure may be measured by sequence analysis software, such as a computer program BLAST using a default parameter, particularly BLASTP or TBLASTN. The amino acid sequences in the present disclosure are shown in a manner from the end N to C.

As mentioned above, the monoclonal antibody according to the present disclosure may be a full-length antibody or may only include functional fragments thereof (such as, Fab, F(ab')2 or scFv fragments), or may be modified to affect the function. The antibody according to the present disclosure includes an anti-MICA antibody with a modified glycosylation mode. In some applications, performing modification to remove unexpected glycosylation sites may be useful, or a fucose part does not exist on the oligosaccharide chain, for instance, to enhance antibody dependent cellular cytotoxicity (ADCC). In some other applications, galactosylation modification may be performed to change complement-dependent cytotoxcity (CDC).

In the present disclosure, the "full-length antibody" is of a tetrapeptide chain structure formed by linking two identical light chains and two identical heavy chains through interchain disulfide bonds, such as immunoglobulin G (IgG), immunoglobulin A (IgA), immunoglobulin M (IgM), immunoglobulin D (IgD) or immunoglobulin E (IgE). The immunoglobulin of the same class may also be divided into different subclasses according to amino acid components, such as IgG1, IgG2, IgG3, and IgG4. The light chain of the immunoglobulin is divided into κ chain or λ chain according to different constant regions.

The term "functional fragment" used in the present disclosure particularly refers to a fragment of an antibody, such as a CDR transplant antibody, Fab, Fab', (Fab')2, Fv or scFv, a nanobody, or any fragment, which can prolong half-life through chemical modification or by being doped into lipidosome. The chemical modification, for instance, refers to addition of poly(alkylene) glycol, such as polyethylene glycol ("pegylation, PEGylation") (pegylation fragment called Fv-PEG, scFv-PEG, Fab-PEG, F(ab')2-PEG or Fab'-PEG) ("PEG" is the polyethylene glycol); and the fragment has MICA binding activity. Preferably, the functional fragment is composed of partial sequences in a heavy or light chain variable region of a source antibody or includes the partial sequences. The partial sequences are enough to remain binding specificity identical to the source antibody and sufficient affinity, for MICA, preferably at least equal to 1/100 of the affinity of the source antibody, at least equal to 1/10 in a more preferred mode. The functional fragment includes at least 3 amino acids, preferably 5, 10, 15, 25, 50, and 100 continuous amino acids in the sequence of the source antibody.

In the present disclosure, unless contrary statement is given, the used term "antigen-binding fragment" generally refers to an antigen binding antibody fragment, and it may include one part of a complete antibody, generally an antigen binding region, or a variable region, for example, including a CDR transplant antibody, Fab, Fab', F(ab')2, Fv or scFv, nanobody, etc.

In the present disclosure, the term "CDR transplant antibody" refers to transplant of CDR of a monoclonal antibody of one species to a variable region of an antibody of another species. For example, CDR of a murine monoclonal antibody may be transplanted to a variable region of a humanized antibody to replace the CDR of the humanized antibody, so that the humanized antibody obtains antigen binding specificity of the murine monoclonal antibody, thereby decreasing heterology of the antibody.

In the present disclosure, the term "Fab antibody" or "Fab" generally refers to an antibody including Fab molecules only, and is composed of VH and CH1 of a heavy chain and a complete light chain, wherein the light chain and the heavy chain are linked through disulfide bonds.

In the present disclosure, the term "nanobody" (a single domain antibody or a VHH antibody) is originally described as an antigen binding immunoglobulin (variable) domain of a "heavy chain antibody" (that is, "an antibody lack of a light chain") (Hamers-Casterman C, AtarhouchT, Muyldermans S, Robinson G, Hamers C, Songa EB, Bendahman N, Hamers R.: "Naturally occurring antibodies devoid of light chains"; Nature 363 ,446-448(1993)), only includes a heavy chain variable region (VH) and conventional CH2 and CH3 regions, and is specifically bound to an antigen through the heavy chain variable region.

In the present disclosure, the term "Fv antibody" generally refers to an antibody formed by linking a light chain variable region (VL) and a heavy chain variable region (VH) through non-covalent bonds, and is the minimum functional fragment of an antibody molecule that remains a complete antigen binding site.

In the present disclosure, the term "single chain antibody" or "scFv" is a fragment formed by linking a heavy chain variable region and a light chain variable region of the antibody through a short chain polypeptide.

In the present disclosure, the term "effective quantity" or "effective dose" refers to a quantity that may produce functions or activity to humans and/or animals and may be accepted by the humans and/or animals.

In the present disclosure, the "pharmaceutically acceptable" component is a substance that is applicable to humans and/or mammals without excessive adverse side effects (such as, toxicity, irritation and allergy), that is, having a reasonable benefit/risk ratio. The term "pharmaceutically acceptable carrier" refers to a carrier used for therapeutic agent administration, including various excipients and diluents.

The medicament according to the present disclosure includes active ingredients of the present disclosure in a safe effective dose and pharmaceutically acceptable carriers. These carriers include (but not limited to): saline, buffer solution, glucose, water, glycerin, ethanol and combinations thereof. A general pharmaceutical preparation should be matched with an administration mode. The administration mode may refer to oral administration, intranasal administration, endermic medication, subcutaneous administration, intramuscular administration or intravenous administration, or intraperitoneal administration. Dosage forms of the medicament according to the present disclosure include injection, oral preparation (tablets, capsules and oral liquid), transdermal agents, and sustained-release agents. For example, the medicament is prepared with normal saline or an aqueous solution containing glucose and other adjuvants by a conventional method. The medicament is suitable to be prepared under a sterile condition. The antibody or the antigen-binding fragment may be applied via intravenous infusion or injection or intramuscular or subcutaneous injection.

In a preferred implementation of the present disclosure, in order to further increase biological acceptability of the antibody, the antibody may be humanized, that is, the antibody is a chimeric antibody or a humanized antibody. The term "chimeric antibody" refers to a recombinant antibody obtained by substituting a constant region amino acid sequence of a monoclonal antibody from one species (such as mouse) into a constant region of an antibody from another species (such as human) by utilizing a recombinant DNA technology. The term "humanized antibody" refers to a recombinant antibody obtained by substituting total non-CDR (Fv framework region (FR)) amino acid sequences in a constant region and a variable region of a monoclonal antibody from one species (such as mouse) into non-CDR amino acid sequences of a constant region and a variable region of an antibody from another species (such as human) by utilizing the recombinant DNA technology. That is, when the constant region of one antibody is humanized, the antibody is refer to as the chimeric antibody. After the total non-CDR amino acid sequences in the constant region and the variable region are humanized, the antibody is called the humanized antibody. The humanized method may be conducted by referring to a conventional antibody engineering technology, which is not described in detail herein.

In the present disclosure, antibody affinity maturation according to the present disclosure refers to a normally present immune function status of the body. In humoral immunity, average affinity of the antibody produced by secondary response is higher than that produced by primary immune response. Such a phenomenon is referred to as the antibody affinity maturation. The function status of the body is a result caused by long-term evolution and continuous adaptation to the external environment, and is of great significance on body defense and maintenance of autoimmune surveillance. An *in-vitro* antibody affinity maturation technology is used in the present disclosure. Specifically, the antibody affinity maturation is a process of mutating CDR amino acids of antibodies by utilizing a molecular biological technique, screening antibodies from an antibody library after mutation, and obtaining an antibody with significantly increased affinity.

Amino acid or nucleic acid sequences involved in the present disclosure are specifically listed in Table 1.

**[Table 1]**

| SEQ ID NO: | Amino acid sequence | Description |
|---|---|---|
| 1 | GFSLTTYGG | Heavy chain variable region CDR1 (HCDR1) of antibody h5A1002 |
| 2 | IWTDGWT | Heavy chain variable region CDR2 (HCDR2) of antibody h5A1002 |
| 3 | ARKGHGYYYAMDY | Heavy chain variable region CDR3 (HCDR3) of antibody h5A1002 |
| 4 | SSVSSSY | Light chain variable region CDR1 (LCDR1) of antibody h5A1002 |
| 5 | STSNLI | Light chain variable region CDR2 (LCDR2) of antibody h5A1002 |
| 6 | HQYHRSPFT | Light chain variable region CDR3 (LCDR3) of antibody h5A1002 |
| 7 | QVQLQESGPGLVKPSETLSLTCTVSGFSLTTY GGHWIRQPPGKGLEWIGMIWTDGWTDYNA AFISRVTISVDTSKNQFSLKLSSVTAADTAVY YCARKGHGYYYAMDYWGQGTLVTVSS | Heavy chain variable region of antibody h5A1002 |
| 8 | EIVLTQSPGTLSLSPGERATLSCTASSSVSSSYL HWYQQKPGQAPRLLIYSTSNLISGIPDRFSGS GSGTDFTLTISRLEPEDFAVYYCHQYHRSPFT FGQGTKLEIK | Light chain variable region of antibody h5A1002 |
| 9 | QVQLQESGPGLVKPSETLSLTCTVSGFSLTTY GVHWIRQPPGKGLEWIGMIWTDGTTDYNAA FISRVTISVDTSKNQFSLKLSSVTAADTAVYY CARKGHGYYYAMDYWGQGTLVTVSS | Heavy chain variable region of antibody h5A1 |
| 10 | EIVLTQSPGTLSLSPGERATLSCTASSSVSSSYL HWYQQKPGQAPRLLIYSTSNLASGIPDRFSGS GSGTDFTLTISRLEPEDFAVYYCHQYHRSPFT FGQGTKLEIK | Light chain variable region of antibody h5A1 |
| 11 | QVQLQESGPGLVKPSETLSLTCTVSGFSLTTY GVHWIRQPPGKGLEWIGMIWTDGTTDYNAA FISRVTISVDTSKNQFSLKLSSVTAADTAVYY CARKGHGYYYAMDYWGQGTLVTVSSASTK GPSVFPLAPSSKSTSGGTAALGCLVKDYFPEP VTVSWNSGALTSGVHTFPAVLQSSGLYSLSSV VTVPSSSLGTQTYICNVNHKPSNTKVDKKVE PKSCDKTHTCPPCPAPELLGGPSVFLFPPKPK | Heavy chain sequence of antibody h5A1- hIgG1 |
| | DTLMISRTPEVTCVVVDVSHEDPEVKFNWY VDGVEVHNAKTKPREEQYNSTYRVVSVLTV LHQDWLNGKEYKCKVSNKALPAPIEKTISKA KGQPREPQVYTLPPSREEMTKNQVSLTCLVK GFYPSDIAVEWESNGQPENNYKTTPPVLDSD GSFFLYSKLTVDKSRWQQGNVFSCSVMHEAL HNHYTQKSLSLSPGK | |
| 12 | EIVLTQSPGTLSLSPGERATLSCTASSSVSSSYL HWYQQKPGQAPRLLIYSTSNLASGIPDRFSGS GSGTDFTLTISRLEPEDFAVYYCHQYHRSPFT FGQGTKLEIKRTVAAPSVFIFPPSDEQLKSGTA SVVCLLNNFYPREAKVQWKVDNALQSGNSQ ESVTEQDSKDSTYSLSSTLTLSKADYEKHKV YACEVTHQGLSSPVTKSFNRGEC | Light chain sequence of antibody h5A1- hIgG1 |
| 13 | QVQLQESGPGLVKPSETLSLTCTVSGFSLTTY GGHWIRQPPGKGLEWIGMIWTDGWTDYNA AFISRVTISVDTSKNQFSLKLSSVTAADTAVY YCARKGHGYYYAMDYWGQGTLVTVSSAST KGPSVFPLAPSSKSTSGGTAALGCLVKDYFPE PVTVSWNSGALTSGVHTFPAVLQSSGLYSLSS VVTVPSSSLGTQTYICNVNHKPSNTKVDKKV EPKSCDKTHTCPPCPAPELLGGPSVFLFPPKP KDTLMISRTPEVTCVVVDVSHEDPEVKFNW YVDGVEVHNAKTKPREEQYNSTYRVVSVLT VLHQDWLNGKEYKCKVSNKALPAPIEKTISK AKGQPREPQVYTLPPSREEMTKNQVSLTCLV KGFYPSDIAVEWESNGQPENNYKTTPPVLDS DGSFFLYSKLTVDKSRWQQGNVFSCSVMHE ALHNHYTQKSLSLSPGK | Heavy chain sequence of antibody h5A1002-hIgG1 |
| 14 | EIVLTQSPGTLSLSPGERATLSCTASSSVSSSYL HWYQQKPGQAPRLLIYSTSNLISGIPDRFSGS GSGTDFTLTISRLEPEDFAVYYCHQYHRSPFT FGQGTKLEIKRTVAAPSVFIFPPSDEQLKSGTA SVVCLLNNFYPREAKVQWKVDNALQSGNSQ ESVTEQDSKDSTYSLSSTLTLSKADYEKHKV YACEVTHQGLSSPVTKSFNRGEC | Light chain sequence of antibody h5A1002 |
| 15 | QVQLQESGPGLVKPSETLSLTCTVSGFSLTTY GGHWIRQPPGKGLEWIGMIWTDGWTDYNA AFISRVTISVDTSKNQFSLKLSSVTAADTAVY YCARKGHGYYYAMDYWGQGTLVTVSSAST KGPSVFPLAPSSKSTSGGTAALGCLVKDYFPE PVTVSWNSGALTSGVHTFPAVLQSSGLYSLSS VVTVPSSSLGTQTYICNVNHKPSNTKVDKKV EPKSCDKTHTCPPCPAPEAAGGPSVFLFPPKP KDTLMISRTPEVTCVVVDVSHEDPEVKFNW YVDGVEVHNAKTKPREEQYNSTYRVVSVLT VLHQDWLNGKEYKCKVSNKALPAPIEKTISK AKGQPREPQVYTLPPSREEMTKNQVSLTCLV KGFYPSDIAVEWESNGQPENNYKTTPPVLDS DGSFFLYSKLTVDKSRWQQGNVFSCSVMHE ALHNHYTQKSLSLSPGK | Heavy chain sequence of antibody h5A1002-hIgG1LALA |
| 16 | QVQLQESGPGLVKPSETLSLTCTVSGFSLTTY GGHWIRQPPGKGLEWIGMIWTDGWTDYNA AFISRVTISVDTSKNQFSLKLSSVTAADTAVY YCARKGHGYYYAMDYWGQGTLVTVSSAST KGPSVFPLAPSSKSTSGGTAALGCLVKDYFPE PVTVSWNSGALTSGVHTFPAVLQSSGLYSLSS | Heavy chain sequence of antibody h5A1002-hIgG1DLE |
| | VVTVPSSSLGTQTYICNVNHKPSNTKVDKKV EPKSCDKTHTCPPCPAPELLGGPDVFLFPPKP KDTLMISRTPEVTCVVVDVSHEDPEVKFNW YVDGVEVHNAKTKPREEQYNSTYRVVSVLT VLHQDWLNGKEYKCKVSNKALPLPEEKTIS KAKGQPREPQVYTLPPSREEMTKNQVSLTCL VKGFYPSDIAVEWESNGQPENNYKTTPPVLD SDGSFFLYSKLTVDKSRWQQGNVFSCSVMHE ALHNHYTQKSLSLSPGK | |
| 17 | QVQLVESGGGLVKPGGSLRLSCAASGFTFSN YAMSWIRQAPGKGLEWVSYISPGGDYIYYAD SVKGRFTISRDNAKNSLYLQMNSLRAEDTAV YYCTTDRRHYGSYAMDYWGQGTLVTVSSAS TKGPSVFPLAPSSKSTSGGTAALGCLVKDYFP EPVTVSWNSGALTSGVHTFPAVLQSSGLYSLS SVVTVPSSSLGTQTYICNVNHKPSNTKVDKK VEPKSCDKTHTCPPCPAPELLGGPSVFLFPPK PKDTLMISRTPEVTCVVVDVSHEDPEVKFNW YVDGVEVHNAKTKPREEQYNSTYRVVSVLT VLHQDWLNGKEYKCKVSNKALPAPIEKTISK AKGQPREPQVYTLPPSREEMTKNQVSLTCLV KGFYPSDIAVEWESNGQPENNYKTTPPVLDS DGSFFLYSKLTVDKSRWQQGNVFSCSVMHE ALHNHYTQKSLSLSPGK | Heavy chain sequence of antibody CLN-619 |
| 18 | DIVMTQSPLSLPVTPGEPASISCRSSKSLLHSN LNTYLYWFLQKPGQSPQILIYRMSNLASGVP DRFSGSGSGTAFTLKISRVEAEDVGVYYCMQ HLEYPFTFGPGTKLEIKRTVAAPSVFIFPPSDE QLKSGTASVVCLLNNFYPREAKVQWKVDNA LQSGNSQESVTEQDSKDSTYSLSSTLTLSKAD YEKHKVYACEVTHQGLSSPVTKSFNRGEC | Light chain sequence of antibody CLN-619 |
| 19 | EIQLVQSGAEVKKPGASVKVSCKASGYAFTS QNIYWVRQAPGQGLEWIGYIEPYNVVPMYN PKFKGRATLTVDKSTSTAYLELSSLRSEDTAV YYCARSGSSNFDYWGQGTLVTVSSASTKGPS VFPLAPSSKSTSGGTAALGCLVKDYFPEPVTV SWNSGALTSGVHTFPAVLQSSGLYSLSSVVTV PSSSLGTQTYICNVNHKPSNTKVDKKVEPKS CDKTHTCPPCPAPELLGGPSVFLFPPKPKDTL MISRTPEVTCVVVDVSHEDPEVKFNWYVDG VEVHNAKTKPREEQYNSTYRVVSVLTVLHQ DWLNGKEYKCKVSNKALPAPIEKTISKAKGQ PREPQVYTLPPSREEMTKNQVSLTCLVKGFY PSDIAVEWESNGQPENNYKTTPPVLDSDGSFF LYSKLTVDKSRWQQGNVFSCSVMHEALHNH YTQKSLSLSPGK | Heavy chain sequence of antibody 1D5V11 |
| 20 | DIQLTQSPSSLSASVGDRVTITCSASSSISSHYL HWYQQKPGKSPKLLIYRTSNLASGVPSRFSG SGSGTDYTLTISSLQPEDFATYYCQQGSSLPLT FGQGTKVEIKRTVAAPSVFIFPPSDEQLKSGTA SVVCLLNNFYPREAKVQWKVDNALQSGNSQ ESVTEQDSKDSTYSLSSTLTLSKADYEKHKV YACEVTHQGLSSPVTKSFNRGEC | Light chain sequence of antibody 1D5V11 |
| 21 | MGLGPVFLLLAGIFPFAPPGAAAEPHSLRYNL TVLSWDGSVQSGFLTEVHLDGQPFLRCDRQ KCRAKPQGQWAEDVLGNKTWDRETRDLTG NGKDLRMTLAHIKDQKEGLHSLQEIRVCEIH | MICA |
| | EDNSTRSSQHFYYDGELFLSQNLETKEWTMP QSSRAQTLAMNVRNFLKEDAMKTKTHYHA MHADCLQELRRYLKSGVVLRRTVPPMVNVT RSEASEGNITVTCRASGFYPWNITLSWRQDG VSLSHDTQQWGDVLPDGNGTYQTWVATRIC QGEEQRFTCYMEHSGNHSTHPVPSGKVLVL QSHWQTFHVSAVAAAAIFVIIIFYVRCCKKKT SAAEGPELVSLQVLDQHPVGTSDHRDATQLG FQPLMSDLGSTGSTEGA | |
| 22 | RTVPPMVNVTRSEASEGNITVTCRASGFYPW NITLSWRQDGVSLSHDTQQWGDVLPDGNGT YQTWVATRICQGEEQRFTCYMEHSGNHSTHP VPSGKVLVLQSHWPKSCDKTHTCPPCPAPEL LGGPSVFLFPPKPKDTLMISRTPEVTCVVVAV SHEDPEVKFNWYVDGVEVHNAKTKPREEQY ASTYRVVSVLTVLHQDWLNGKEYKCKVSNK ALPAPIEKTISKAKGQPREPQVYTLPPSREEM TKNQVSLTCLVKGFYPSDIAVEWESNGQPEN NYKTTPPVLDSDGSFFLYSKLTVDKSRWQQG NVFSCSVMHEALHNHYTQKSLSLSPGK | MICA002α3-Fc |
| 23 | RRVPPMVNVTRSEASEGNITVTCRASSFYPR NITLTWRQDGVSLSHDTQQWGDVLPDGNGT YQTWVATRICQGEEQRFTCYMEHSGNHSTHP VPSGKVLVLQSHWPKSCDKTHTCPPCPAPEL LGGPSVFLFPPKPKDTLMISRTPEVTCVVVAV SHEDPEVKFNWYVDGVEVHNAKTKPREEQY ASTYRVVSVLTVLHQDWLNGKEYKCKVSNK ALPAPIEKTISKAKGQPREPQVYTLPPSREEM TKNQVSLTCLVKGFYPSDIAVEWESNGQPEN NYKTTPPVLDSDGSFFLYSKLTVDKSRWQQG NVFSCSVMHEALHNHYTQKSLSLSPGK | MICA004α3-Fc |
| 24 | RTVPPMVNVTCSEVSEGNITVTCRASSFYPRN ITLTWRQDGVSLSHNTQQWGDVLPDGNGTY QTWVATRIRQGEEQRFTCYMEHSGNHGTHP VSGKVLVLQSHWPKSCDKTHTCPPCPAPELL GGPSVFLFPPKPKDTLMISRTPEVTCVVVAVS HEDPEVKFNWYVDGVEVHNAKTKPREEQYA STYRVVSVLTVLHQDWLNGKEYKCKVSNKA LPAPIEKTISKAKGQPREPQVYTLPPSREEMT KNQVSLTCLVKGFYPSDIAVEWESNGQPENN YKTTPPVLDSDGSFFLYSKLTVDKSRWQQGN VFSCSVMHEALHNHYTQKSLSLSPGK | MICA005α3-Fc |
| 25 | RTVPPMVNVTRSEASEGNITVTCRASSFYPRN IILTWRQDGVSLSHDTQQWGGVLPDGNGTY QTWVATRICRGEEQRFTCYMEHSGNHSTHPV PSGKVLVLQSHWPKSCDKTHTCPPCPAPELL GGPSVFLFPPKPKDTLMISRTPEVTCVVVAVS HEDPEVKFNWYVDGVEVHNAKTKPREEQYA STYRVVSVLTVLHQDWLNGKEYKCKVSNKA LPAPIEKTISKAKGQPREPQVYTLPPSREEMT KNQVSLTCLVKGFYPSDIAVEWESNGQPENN YKTTPPVLDSDGSFFLYSKLTVDKSRWQQGN VFSCSVMHEALHNHYTQKSLSLSPGK | MICA008α3-Fc |
| 26 | RTVPPMVNVTCSEVSEGNITVTCRASSFYPRN ITLTWRQDGVSLSHNTQQWGDVLPDGNGTY QTWVATRIRQGEEQRFTCYMEHSGNHGTHP VPSGKVLVLQSQRPKSCDKTHTCPPCPAPELL | MICB001α3-Fc |
| | GGPSVFLFPPKPKDTLMISRTPEVTCVVVAVS HEDPEVKFNWYVDGVEVHNAKTKPREEQYA STYRVVSVLTVLHQDWLNGKEYKCKVSNKA LPAPIEKTISKAKGQPREPQVYTLPPSREEMT KNQVSLTCLVKGFYPSDIAVEWESNGQPENN YKTTPPVLDSDGSFFLYSKLTVDKSRWQQGN VFSCSVMHEALHNHYTQKSLSLSPGK | |
| 27 | RTVPPMVNVTCSEVSEGNITVTCRASSFYPRN ITLTWRQDGVSLSHNTQQWGDVLPDGNGTY QTWVATRIRQGEEQRFTCYMEHSGNHGTHP VPSGKVLALQSQWPKSCDKTHTCPPCPAPEL LGGPSVFLFPPKPKDTLMISRTPEVTCVVVAV SHEDPEVKFNWYVDGVEVHNAKTKPREEQY ASTYRVVSVLTVLHQDWLNGKEYKCKVSNK ALPAPIEKTISKAKGQPREPQVYTLPPSREEM TKNQVSLTCLVKGFYPSDIAVEWESNGQPEN NYKTTPPVLDSDGSFFLYSKLTVDKSRWQQG NVFSCSVMHEALHNHYTQKSLSLSPGK | MICB005α3-Fc |

The embodiments of the present disclosure are described below in detail. The embodiments described below are illustrative, merely used for explaining the present disclosure, but they cannot be understood as a limitation to the present disclosure. Procedures in the embodiments without indicated specific technologies or conditions are conducted according to technologies or conditions described in the document in the art or according to the product manual. Used reagents or instruments without indicating manufacturers are all conventional products purchasable in the market.

### Example 1: Production of antibody

Specific experimental operations of production of the antibody are as follows: (1) ExpiCHO cells (purchased from Thermo Fisher) were cultured by utilizing an ExpiCHO Expression Medium (purchased from Thermo Fisher), and a cell concentration was regulated as 6×10⁶/mL to obtain an ExpiCHO cell solution; (2) a vector pcDNA3.4 containing an antibody heavy chain and an antibody light chain (synthesized by commissioned GenScript, Nanjing) was added into 2 mL of OptiSFM medium (purchased from Thermo Fisher) according to a ratio of 1:1 to obtain a solution A; (3) 160 µl of an ExpiFectamineCHO transfection reagent (purchased from Thermo Fisher) was added into the 2 mL OptiSFM medium (purchased from Thermo Fisher) to obtain a solution B; (4) then the solution A and the solution B were mixed to obtain a transfection mixture, and the transfection mixture was completely added into 50 mL of the ExpiCHO cell solution within 5 minutes; (5) the solution was cultured under conditions of 37°C and 5% CO₂ for 1 day, 8 mL of Feed and 300 µl of Enhancer (purchased from Thermo Fisher) were added into the solution, and the cultural supernatant was harvested within 9 days after culture of the solution under the conditions of 37°C and 5% CO₂, wherein the 8 mL of Feed was added on Day 5; and (6) affinity purification was performed on the cultural supernatant by utilizing a Protein A purification column (purchased from Nano-Micro), thereby obtaining a maternal humanized antibody h5A1.

### Example 2: Antibody affinity maturation

In an affinity maturation process of a natural antibody, somatic hypermutation was mainly concentrated in a CDR region. Through *in-vitro* experiments, single point saturation mutation was performed on each site of the CDR region to obtain sufficient mutation diversity, while a protein structure was not destroyed. Such a route can realize *in-vitro* reproduction of the somatic hypermutation of the natural antibody *in vivo* with the highest similarity.

Each amino acid site of the CDR region was subjected to the single point saturation mutation to construct a deviation-free single point saturation mutation plasmid library of the maternal antibody. Mutation sites with enhanced specific binding to an antigen were screened out with ELISA. Then, these sites were subjected to combinatorial screening to obtain candidate antibody mutation sequences.

Through the technical route, the maternal humanized antibody h5A1 (prepared by the method in Example 1, including a heavy chain variable region as set forth in SEQ ID NO: 9 and a light chain variable region as set forth in SEQ ID NO: 10, as known from a patent CN114369162A), was subjected to affinity maturation to obtain a high-affinity MICA monoclonal antibody h5A1002 (prepared by the method in Example 1) including sequences of a heavy chain variable region (SEQ ID NO: 7) and a light chain variable region (SEQ ID NO: 8). Comparison of CDR sequences of the maternal humanized antibody h5A1 and the affinity maturation antibody h5A1002 is shown as Table 2.

**[Table 2]**

| | HCDR1 | HCDR2 | HCDR3 | LCDR1 | LCDR2 | LCDR3 |
|---|---|---|---|---|---|---|
| h5A1 | GFSLTTYG*V* | IWTDG*T*T | ARKGHGYYYAMDY | SSVSSSY | STSNL*A* | HQYHRSPFT |
| h5A1002 | GFSLTTYG*G* | IWTDG*W*T | ARKGHGYYYAMDY | SSVSSSY | STSNL*I* | HQYHRSPFT |

### Example 3: Affinity assay of antibody

Biacore, as a method for analyzing biomolecular interaction based on an optical surface plasmon resonance (SPR), can not only detect specific binding between an antigen and an antibody, but also obtain very important data in research and development of medicaments, such as a binding rate constant (Ka) among molecules, a dissociation rate constant (Kd), and an equilibrium dissociation constant (KD), thereby calculating the affinity of the antibody.

In a Biacore 8K (Cytiva) system, the antibody was diluted to a concentration of 10 µg/mL with running buffer (HBS-EP), and the antibody was conjugated onto a protein A (Cytiva, 29127556) chip at a flow rate of 10 µl/min. Kinetics and affinity data of the binding of the antigen and the antibody were detected at a flow rate of 30 µl/min; binding period was 120s; and dissociation time was 800s.

Kinetic and affinity data of binding of the maternal antibody h5A1 and the affinity maturation antibody h5A1002 and MICA were detected. Results are shown as Table 3. Compared with the maternal antibody h5A1, the affinity of the affinity maturation antibody h5A1002 to MICA is greatly increased by about 39 times.

**[Table 3]**

| Ab | Ka (1/Ms) | Kd (1/s) | KD (M) |
|---|---|---|---|
| h5A1 | 2.97E+03 | 6.41E-04 | 2.15E-07 |
| h5A1002 | 2.38E+04 | 1.29E-04 | 5.40E-09 |

| | | | |
|---|---|---|---|
| Notes: Ka represents a binding rate constant (the larger the value is, the higher the affinity is); Kd represents a dissociation rate constant (the smaller the number is, the higher the affinity is), reflecting an affinity of a compound to a target; and KD represents Kd/Ka, which is the equilibrium dissociation constant (an affinity constant), wherein the smaller the KD is, the less the dissociation is, and the higher the affinity is. | | | |

### Example 4: ELISA binding assay of MICA antibodies

(1) ELISA was used to detect binding characteristics of the maternal antibody h5A1, the affinity maturation antibody h5A1002 and domains α3 of MICA and MICB. Fc fusion proteins in extracellular domains α3 (domains α3 of MICA-002, 005, 008, 004 and MICB-001, 005) of multiple variants of MICA and MICB were coated into a 96-well plate. After the antibodies were added, signal intensity was used for judging the binding characteristics of the antibodies and MICA and MICB.

The fusion proteins (produced in the Applicant's laboratory; an amino acid sequence of MICA002α3-Fc is as set forth in SEQ ID NO: 22; an amino acid sequence of MICA005α3-Fc is as set forth in SEQ ID NO: 24; an amino acid sequence of MICA008α3-Fc is as set forth in SEQ ID NO: 25; an amino acid sequence of MICA004α3-Fc is as set forth in SEQ ID NO: 23; an amino acid sequence of MICB001α3-Fc is as set forth in SEQ ID NO: 26; and an amino acid sequence of MICB005α3-Fc is as set forth in SEQ ID NO: 27) were diluted to a concentration of 1 µg/ml with PBS and then added into a 96-well plate in a volume of 100 µl/well. The plate was placed at 4°C overnight. The PBS in the 96-well plate was removed by sucking. After the plate was washed with PBST (that is, PBS having a pH value of 7.2 included 0.1 volume% of Tween 20) for 6 times, PBS containing 10% of BSAwas added in a volume of 200 µl/well. The plate was incubated at 37°C for 2 hours for blocking. The blocking solution was removed. After the plate was washed with PBST for 6 times, an antibody h5A1 (having a heavy chain sequence as set forth in SEQ ID NO: 11 and a light chain sequence as set forth in SEQ ID NO: 12), an antibody h5A1002 (having a heavy chain sequence as set forth in SEQ ID NO: 13 and a light chain sequence as set forth in SEQ ID NO: 14), and control IgG1 (purchased from Biointron), which were gradient-diluted (at a highest working concentration of 20,000 ng/ml, diluted by 5 times, 8 gradients) with PBST containing 0.05% of BSA, were added in a volume of 100 µl/well, and the plate was incubated at 37°C for 1 hour. A reaction system in the wells was removed by sucking. After the plate was washed with PBST for 6 times, a horse radish peroxidase (HRP) labeled anti-human secondary antibody (Fab-specific) (purchased from Sigma) diluted with the PBST containing 0.05% of BSA was added in a volume of 100 µl/well. The plate was incubated at 37°C for 1 hour. The secondary antibody in the wells was removed by sucking. After the plate was washed with PBST for 6 times, tetramethyl benzidine (TMB) was added in a volume of 80 µl/well, and the plate was incubated at room temperature for 3 min. 4M sulfuric acid was added in a volume of 80 µl/well to terminate the reaction. An absorbance value was read by a microplate reader at 450 mm.

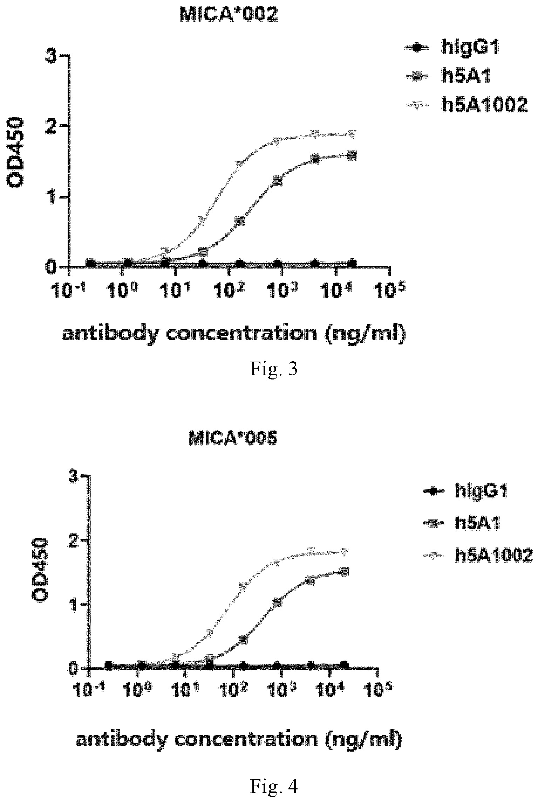

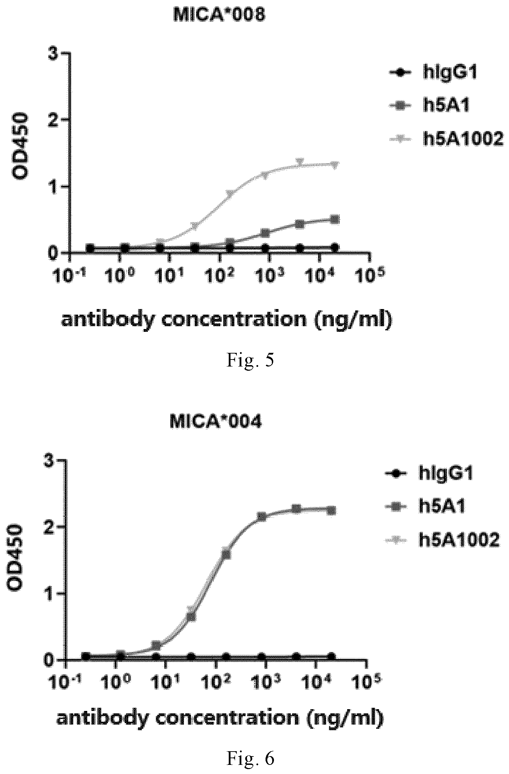

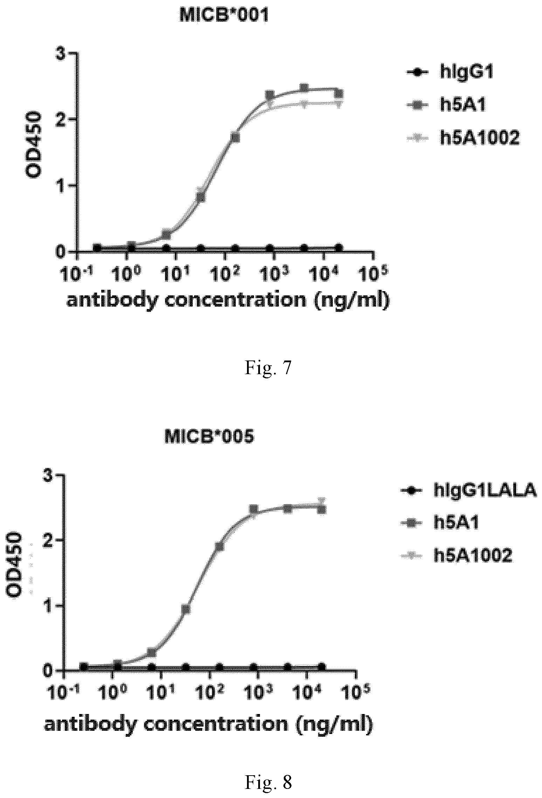

The results are as shown in Fig. 3, Fig. 4, Fig. 5, Fig. 6, Fig. 7, and Fig. 8. It is indicated that, the antibody according to the present disclosure can bind to MICA*002, MICA*005, MICA*008, MICA*004, MICB*001, and MICB*005, and binding of the affinity maturation antibody h5A1002 to the MICA*002, MICA*005, and MICA*008 is stronger than that of the maternal humanized antibody h5A1.

(2) Binding characteristics of the affinity maturation antibody h5A1002, homogeneous antibodies CLN-619 (Cullinan, having a sequence from a patent US20210253711A1, prepared by the method in embodiment 1) and 1D5V11 (Genentech, having a sequence known from a patent US20200055939A1, prepared by the method in Example 1) and the domains α3 of MICA and MICB are detected by utilizing ELISA. Fc fusion proteins in extracellular domains α3 (domains α3 of MICA-002, 005, 008, 004 and MICB-001, 005) of multiple variants of MICA and MICB were coated into 96-well plate. After the antibodies were added, signal intensity was used for evaluating the binding characteristics of the antibodies and MICA and MICB.

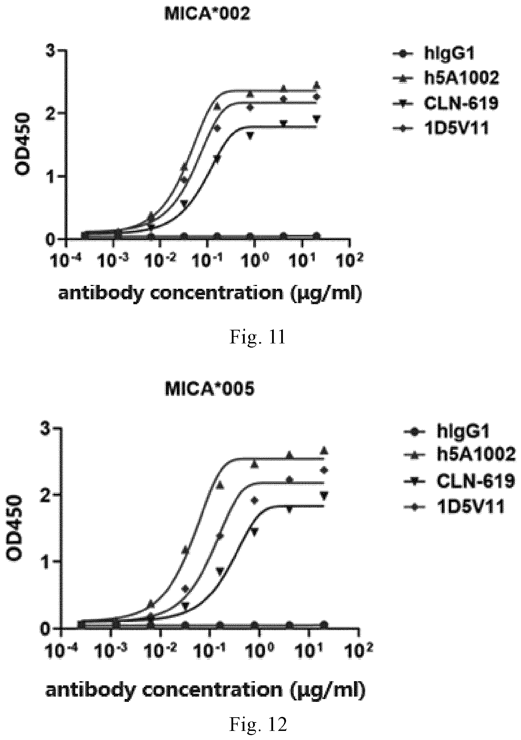

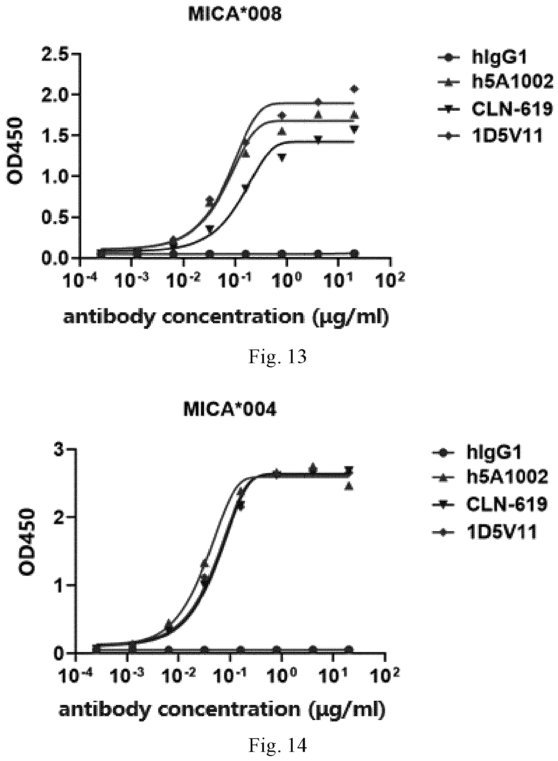

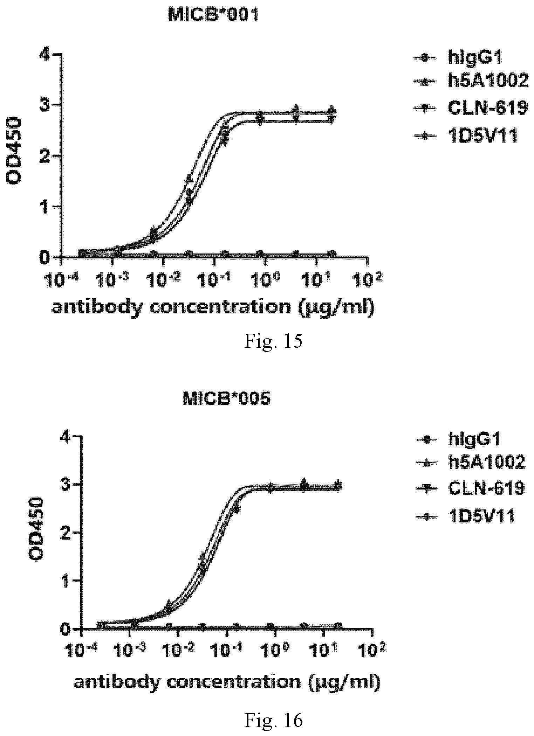

Experimental procedures are described as in step (1), and the results are as shown in Fig. 11, Fig. 12, Fig. 13, Fig. 14, Fig. 15, and Fig. 16. The results indicate that the antibody according to the present disclosure can bind to MICA*002, MICA*005, MICA*008, MICA*004, MICB*001, and MICB*005, and that binding of the affinity maturation antibody h5A1002 to the MICA*002, MICA*005 and MICA*008 is stronger than that of antibody CLN-619 (Cullinan) to the identical target.

### Example 5: Flow cytometry binding assay of MICA antibodies

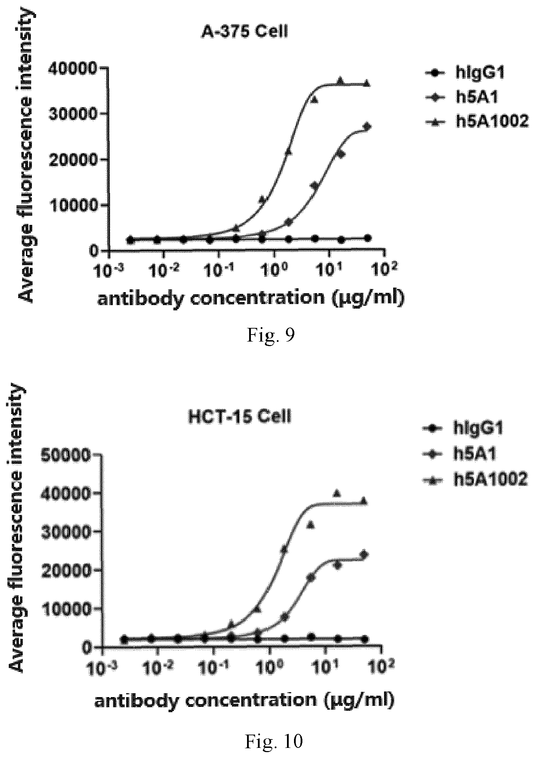

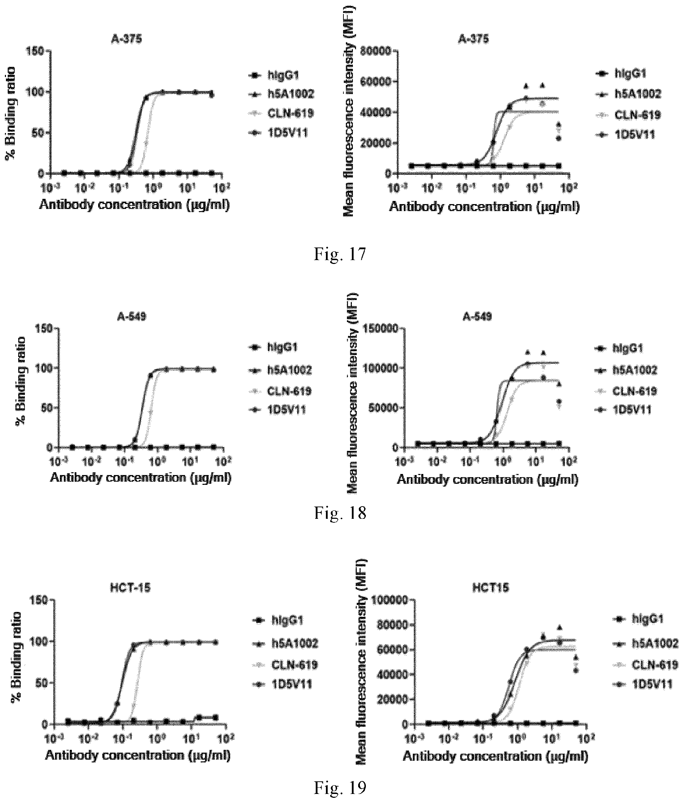

(1) Tumor cells were diluted to a density of 2×10⁶/mL with PBS and then added into a 1.5 ml EP tube in a volume of 100 µl/tube. 10 µl/tube of goat serum was added into the EP tube; and blocking was conducted at 4°C for 30 min. Antibody h5A1 (having a heavy chain sequence as set forth in SEQ ID NO: 11 and a light chain sequence as set forth in SEQ ID NO: 12), antibody h5A1002 (having a heavy chain sequence as set forth in SEQ ID NO: 13 and a light chain sequence as set forth in SEQ ID NO: 14), and control IgG1 (purchased from Biointron) were gradient-diluted (at a highest working concentration of 50 µg/ml, 10 gradients, diluted by 3 times for each gradient) and added. The tube was incubated at 4°C for 30 min. 1 mL of PBS was added into the EP tube, and centrifugation was conducted 4°C at a rate of 3,500 rpm for 5min. The supernatant was removed. The EP tube was washed once with the PBS. The supernatant was removed after centrifugation. The cells were resuspended with PBS in a volume of 100 µl/tube. An Alexa-647 labeled goat anti-human secondary antibody (purchased from Jackson lab) was added into the tube in a volume of 1 µl/tube; and the plate was incubated in the dark at 4°C for 30 min. The tube was washed twice with PBS, and the supernatant was removed after centrifugation. The cells were resuspended with the PBS in a volume of 200 µl/tube, and detection was conducted by a flow cytometer. The results are as shown in Fig. 9 and Fig. 10. The results indicate that binding of the affinity maturation antibody h5A1002 according to the present disclosure to melanoma cells A-375 and colorectal cancer cells HCT-15 is stronger than that of the maternal humanized antibody h5A1.
(2) Tumor cells were diluted to a density of 2×10⁶/mL with PBS and added into a 1.5 ml EP tube in a volume of 100 µl/tube. 10 µl/tube of goat serum was added into the EP tube; and blocking was conducted at 4°C for 30 min. Antibody h5A1002 (having a heavy chain sequence as set forth in SEQ ID NO: 13 and a light chain sequence as set forth in SEQ ID NO: 14), antibody CLN-619 (having a heavy chain sequence as set forth in SEQ ID NO: 17 and a light chain sequence as set forth in SEQ ID NO: 18), an antibody 1D5V11 (having a heavy chain sequence as set forth in SEQ ID NO: 19 and a light chain sequence as set forth in SEQ ID NO: 20), and control IgG1 (purchased from Biointron) were gradient-diluted (at a highest working concentration of 50 µg/ml, 10 gradients, diluted by 3 times for each gradient) and added. The tube was incubated at 4°C for 30 min. 1 mL of PBS was added into the EP tube, and centrifugation was conducted 4°C at a rate of 3,500 rpm for 5min. The supernatant was removed. Then, the EP tube was washed once with the PBS, and the supernatant was removed after centrifugation. The cells were resuspended with PBS in a volume of 100 µl/tube. An Alexa-647 labeled goat anti-human secondary antibody (purchased from Jackson lab) was added into the tube in a volume of 1 µl/tube, and the tube was incubated in the dark at 4°C for 30 min. The tube was washed twice with PBS, and the supernatant was removed after centrifugation. The cells were resuspended with the PBS in a volume of 200 µl/tube, and detection was conducted by a flow cytometer. The results are as shown in Fig. 17, Fig. 18, Fig. 19, and Fig. 20. The results indicate that, binding of the affinity maturation antibody h5A1002 according to the present disclosure to the tumor cells (for example, melanoma cells A-375, non-small cell lung cancer cells A-549, colorectal cancer cells HCT-15 and breast cancer cells MDA-MB-231) is stronger than that of the identical-target antibody CLN-619 (Cullinan).

### Example 6: Epitope competition assay

Flow cytometry was used to detect epitope competition conditions of the affinity maturation antibody h5A1002 and the homogeneous antibodies CLN-619 (Cullinan) and 1D5V11 (Genentech).

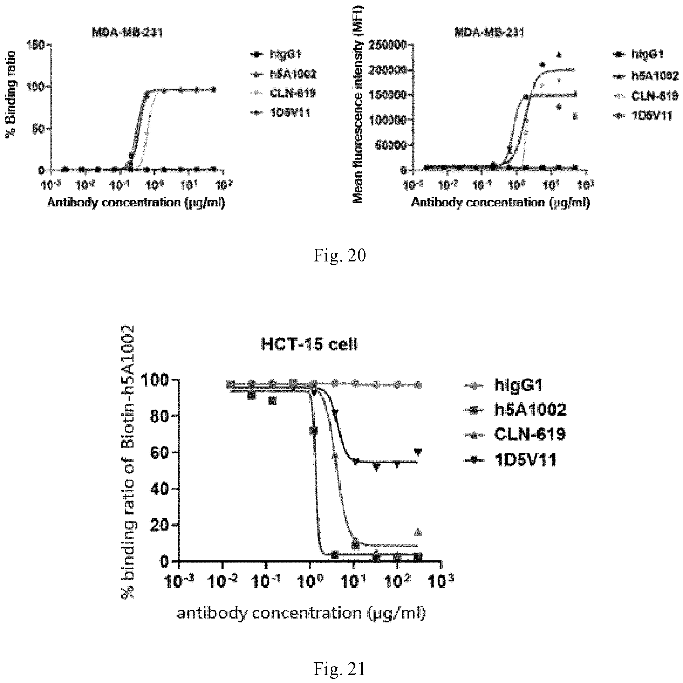

HCT-15 cells were diluted to a density of 2×10⁶/mL with PBS and added into a 1.5 ml EP tube in a volume of 100 µl/tube. 10 µl/tube of goat serum was added into the EP tube, and blocking was conducted at 4°C for 30 min. Antibody h5A1002 (having a heavy chain sequence as set forth in SEQ ID NO: 13 and a light chain sequence as set forth in SEQ ID NO: 14), antibody CLN-619 (having a heavy chain sequence as set forth in SEQ ID NO: 17 and a light chain sequence as set forth in SEQ ID NO: 18), antibody 1D5V11 (having a heavy chain sequence as set forth in SEQ ID NO: 19 and a light chain sequence as set forth in SEQ ID NO: 20), and hIgG1 (purchased from Biointron) were gradient-diluted (at a highest working concentration of 300 µg/ml, 10 gradients, diluted by 3 times for each gradient) and added. The tube was incubated at 4°C for 30 min. A biotin-labeled affinity maturation antibody Biotin-h5A1002 (having a heavy chain sequence as set forth in SEQ ID NO: 13 and a light chain sequence as set forth in SEQ ID NO: 14) was added. The tube was incubated at 4°C for 30 min. 1 mL of PBS was added into the EP tube, and centrifugation was conducted 4°C at a rate of 3,500 rpm for 5min. The supernatant was removed. The EP tube was washed once with the PBS, and the supernatant was removed after centrifugation. The cells were resuspended with PBS in a volume of 100 µl/tube. An Alexa-647 labeled Streptavidin secondary antibody (purchased from Biolegend) was added into the tube in a volume of 1 µl/tube, and the tube was incubated in the dark at 4°C for 30 min. The tube was washed twice with PBS, and the supernatant was removed after centrifugation. The cells were resuspended with the PBS in a volume of 200 µl/tube, and detection was conducted by a flow cytometer. The results are as shown in Fig. 21. The results indicate that, the antigen bound to the affinity maturation antibody h5A1002 according to the present disclosure and the CLN-619 (Cullinan) has epitope overlap, while the h5A1002 and the 1D5V11 (Genentech) are bound to different epitopes.

### Example 7: Endocytosis assay of MICA antibodies

The affinity maturation antibody h5A1002 as well as the homogeneous antibodies CLN-619 (Cullinan) and 1D5V11 (Genentech) were labeled with pHrodo (purchased from Thermo) and added into a cell culture system. After incubation for 24 hours, fluorescence of cells was detected. Intensity of a fluorescence signal reflects endocytosis conditions in the antibodies.

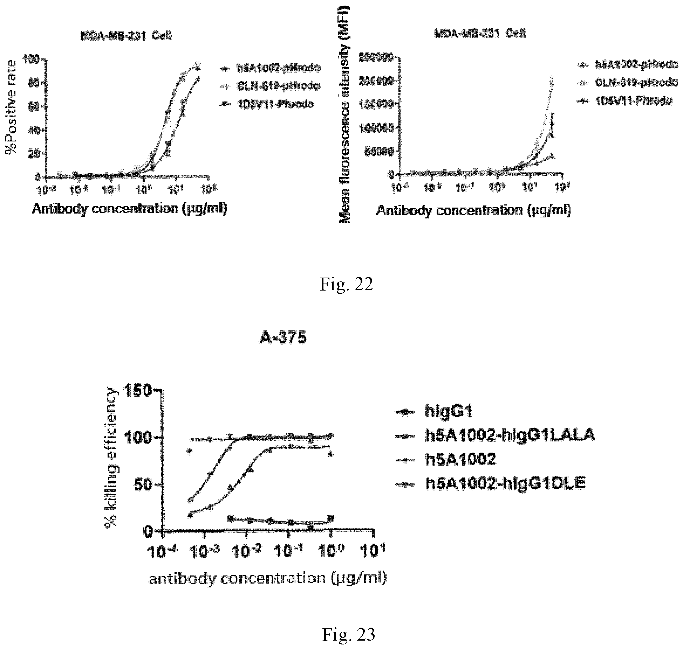

MDA-MB-231 cells were diluted to a density of 1×10⁵/mL with PBS and added into a 96-well plate in a volume of 200 µl/well. h5A1002-pHrodo (having a heavy chain sequence as set forth in SEQ ID NO: 13 and a light chain sequence as set forth in SEQ ID NO: 14), CLN-619-pHrodo (having a heavy chain sequence as set forth in SEQ ID NO: 17 and a light chain sequence as set forth in SEQ ID NO: 18), and 1D5V11-pHrodo (having a heavy chain sequence as set forth in SEQ ID NO: 19 and a light chain sequence as set forth in SEQ ID NO: 20) were gradient-diluted (at a highest working concentration of 50 µg/ml, 10 gradients, diluted by 3 times for each gradient) and added into the 96-well plate. The plate was incubated under conditions of 37°C and 5%CO₂ for 24 hours. Then, endocytosis of the antibodies was detected with flow cytometry. The results are as shown in Fig. 22. The results indicate that, the endocytosis of the affinity maturation antibody h5A1002 according to the present disclosure is weaker than that of the homogeneous antibodies CLN-619 and 1D5V11, suggesting that the affinity maturation antibody h5A1002 has higher drugability as a monoclonal antibody.

### Example 8: Promotion of effect of PBMCs for killing tumor cells by MICA antibodies

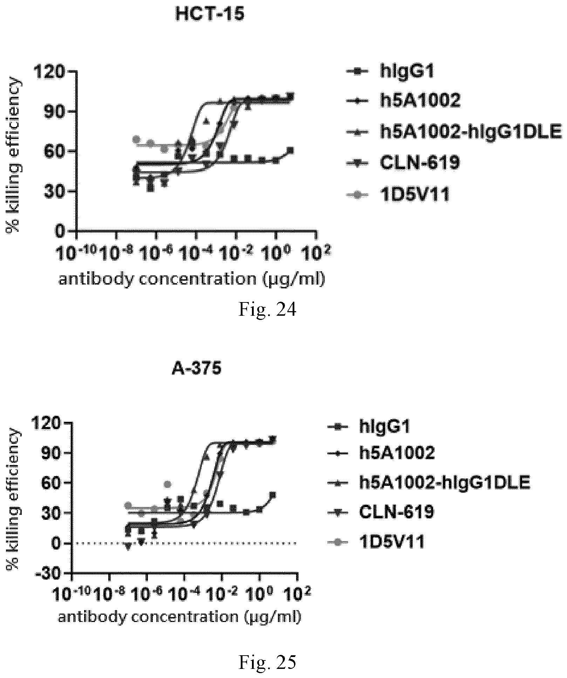

Capability of MICA monoclonal antibody of promoting an effect of PBMCs for killing melanoma cells A-375 and colorectal cancer cells HCT-15 was detected.
(1) A complete medium RPMI-1640 was added into a 16-well RTCA plate according to a volume of 50 µl/well; and calibration was conducted on a machine.
(2) Tumor cells were diluted to a density of 2× 10⁵/mL with the complete medium RPMI-1640. The cells were added into the RTCA plate obtained in the step (1) according to a volume of 50 µl/well. Then, a cell coefficient was detected under conditions of 37°C and 5% CO₂ by using equipment xCELLigence RTCA MP for 24 h.
(3) h5A1002 (having a heavy chain sequence as set forth in SEQ ID NO: 13 and a light chain sequence as set forth in SEQ ID NO: 14), h5A1002-hIgG1LALA (prepared by the method in Example 1; having a heavy chain sequence as set forth in SEQ ID NO: 15 and a light chain sequence as set forth in SEQ ID NO: 14), h5A1002-hIgG1DLE (prepared by the method in Example 1; having a heavy chain sequence as set forth in SEQ ID NO: 16 and a light chain sequence as set forth in SEQ ID NO: 14), antibody CLN-619 (having a heavy chain sequence as set forth in SEQ ID NO: 17 and a light chain sequence as set forth in SEQ ID NO: 18), antibody 1DSV11(having a heavy chain sequence as set forth in SEQ ID NO: 19 and a light chain sequence as set forth in SEQ ID NO: 20), and hIgG1 (purchased from Biointron) were gradient-diluted (the highest concentration in Fig. 23 was 10 µg/ml, 8 gradients, diluted by 3 times for each gradient. In Fig. 24 and Fig. 25, the highest concentration was 50 µg/ml, 12 gradients, diluted by 5 times for each gradient) by using the complete medium RPMI-1640. The antibodies were added into the RTCA plate obtained in the step (2), and an addition volume was 20 µl/well.
(4) PBMCs (purchased from Milestone^{®} Biotechnologies) were diluted to a density of 1.25×10⁶/mL with the complete medium RPMI-1640, and added into the RTCA plate obtained in the step (3) according to an addition volume of 80 µl/well.
(5) The reaction system obtained in the step (4) was placed under conditions of 37°C and 5% CO₂. The cell coefficient was detected by using equipment xCELLigence RTCA MP.

As shown in Fig. 23, the MICA antibody according to the present disclosure promoted the effect of PBMCs for killing tumors; and the hIgG1 subtype antibody h5A1002, the ADCC-free antibody h5A1002-hIgG1LALA and the ADCC-enhanced antibody h5A1002-hIgGIDLE had the promoting effect.

As shown in Fig. 24 and Fig. 25, the antibody h5A1002 according to the present disclosure promoted the effect of PBMCs for killing the tumors. Moreover, the promoted killing effect of the hIgG1 subtype antibody (h5A1002) was better than that of the identical-target antibody CLN-619 (also the hIgG1 subtype); and the ADCC-enhanced antibody h5A1002-hIgGIDLE had the most excellent promoting effect compared with the other antibodies.

In the present description, descriptions of the reference terms such as "one embodiment", "some embodiments", "examples", "specific examples" and "some examples" mean that, specific features, structures, materials or characteristics described in combination with the embodiments or examples are included in at least one embodiment or example of the present disclosure. In the present description, schematic expressions of the above terms are unnecessarily specified at the same embodiment or example. Moreover, the specific features, structures, materials or characteristics described herein may be in combination in any of or more embodiments or examples in an appropriate mode. In addition, in absence of mutual contradiction, different embodiments or examples described in the present description and features of the different embodiments or examples may be in integration and combination by those skilled in the art.

Although the embodiments of the present disclosure have been illustrated and described above, it can be understood that the above embodiments are illustrative and not limitations on the present disclosure. Those skilled in the art can make changes, modifications, replacements and transformations to the above embodiments without departing from the scope of the present disclosure.

## Claims

1. An antibody or antigen-binding fragment thereof, comprising:
a heavy chain variable region CDR1 sequence, a heavy chain variable region CDR2 sequence, and a heavy chain variable region CDR3 sequence as set forth in SEQ ID NO: 1, SEQ ID NO: 2, and SEQ ID NO: 3, respectively, or as set forth in amino acid sequences having at least 80% identity to SEQ ID NO: 1, SEQ ID NO: 2, and SEQ ID NO: 3, respectively; and
a light chain variable region CDR1 sequence, a light chain variable region CDR2 sequence, and a light chain variable region CDR3 sequence as set forth in SEQ ID NO: 4, SEQ ID NO: 5, and SEQ ID NO: 6, respectively, or as set forth in amino acid sequences having at least 80% identity to SEQ ID NO: 4, SEQ ID NO: 5, and SEQ ID NO: 6, respectively.

2. The antibody or the antigen-binding fragment thereof according to claim 1, comprising:
the heavy chain variable region CDR1 sequence as set forth in SEQ ID NO: 1;
the heavy chain variable region CDR2 sequence as set forth in SEQ ID NO: 2;
the heavy chain variable region CDR3 sequence as set forth in SEQ ID NO: 3;
the light chain variable region CDR1 sequence as set forth in SEQ ID NO: 4;
the light chain variable region CDR2 sequence as set forth in SEQ ID NO: 5; and
the light chain variable region CDR3 sequence as set forth in SEQ ID NO: 6.

3. The antibody or the antigen-binding fragment thereof according to claim 1, capable of specifically recognizing a major histocompatibility complex class I chain related polypeptide A (MICA).

4. The antibody or the antigen-binding fragment thereof according to claim 1, wherein the antibody comprises a heavy chain variable region and a light chain variable region, an amino acid sequence of the heavy chain variable region being as set forth in SEQ ID NO: 7, and an amino acid sequence of the light chain variable region being as set forth in SEQ ID NO: 8.

5. The antibody or the antigen-binding fragment thereof according to claim 1, wherein the antibody is a humanized antibody.

6. The antibody or the antigen-binding fragment thereof according to any one of claims 1 to 5, comprising a heavy chain frame region sequence and a light chain frame region sequence, wherein at least one part of at least one of the heavy chain frame region sequence and the light chain frame region sequence is derived from at least one of a murine antibody, a humanized antibody, a primate-derived antibody, or a mutant thereof.

7. The antibody or the antigen-binding fragment thereof according to any one of claims 1 to 5, comprising at least one of a heavy chain constant region and a light chain constant region, wherein at least one part of the at least one of the heavy chain constant region and the light chain constant region is derived from at least one of a humanized antibody, a primate-derived antibody, a murine antibody, or a mutant thereof;
optionally, the light chain constant region and the heavy chain constant region are both derived from:
a murine IgG1 antibody, a murine IgG2a antibody, or a mutant thereof; or
a human IgG1 antibody, a human IgG2 antibody, a human IgG3 antibody, a human IgG4 antibody, or a mutant thereof.

8. The antibody or the antigen-binding fragment thereof according to claim 1, wherein the antibody or antigen-binding fragment thereof is at least one of a single chain antibody, a multimeric antibody, a CDR transplant antibody, a Fab antibody, or an Fv antibody.

9. A nucleic acid molecule, encoding the antibody or antigen-binding fragment thereof according to any one of claims 1 to 8.

10. An expression vector, carrying the nucleic acid molecule according to claim 9.

11. A method for preparing the antibody or antigen-binding fragment thereof according to any one of claims 1 to 8, comprising:
introducing the expression vector according to claim 10 into cells; and
culturing the cells under conditions suitable for protein expression and secretion, to obtain the antibody or antigen-binding fragment thereof,
optionally, the cells are prokaryotic cells or eukaryotic cells; and
optionally, the cells are eukaryotic cells.

12. A recombinant cell, expressing the antibody or antigen-binding fragment thereof according to any one of claims 1 to 8, or carrying the nucleic acid molecule according to claim 9 or the expression vector according to claim 10.

13. A composition, comprising the antibody or antigen-binding fragment thereof according to any one of claims 1 to 8, the nucleic acid molecule according to claim 9, the expression vector according to claim 10, or the recombinant cell according to claim 12.

14. A medicament, comprising: the antibody or antigen-binding fragment thereof according to any one of claims 1 to 8, the nucleic acid molecule according to claim 9, the expression vector according to claim 10, the recombinant cell according to claim 12, or the composition according to claim 13, wherein the medicament is used for preventing and/or treating a MICA-mediated disease,
preferably, the MICA-mediated disease is a cancer;
optionally, the cancer is at least one of lung cancer, liver cancer, ovarian cancer, cervical cancer, skin cancer, bladder cancer, colon cancer, breast cancer, glioma, renal cancer, gastric cancer, esophagus cancer, oral squamous cell carcinoma, and head-and-neck cancer.

15. A kit, comprising the antibody or antigen-binding fragment thereof according to any one of claims 1 to 8, the nucleic acid molecule according to claim 9, the expression vector according to claim 10, or the recombinant cell according to claim 12.
